# EUROPÄISCHE PATENTSCHRIFT

(11) **EP 3 437 468 B1**
(45) Veröffentlichungstag und Bekanntmachung des Hinweises auf die Patenterteilung: **29.09.2021**
(21) Anmeldenummer: 17184291.7
(22) Anmeldetag: 01.08.2017
(51) Int. Cl.: A01K 43/00, G01N 33/08

(54) **VERFAHREN UND VORRICHTUNG ZUM BEARBEITEN EINER KALKSCHALE IM BEREICH DES STUMPFEN POLS EINES VOGELEIS**
METHOD AND DEVICE FOR TREATING A CALCAREOUS CELL IN THE AREA OF THE BLUNT POLE OF AN AVIAN EGG
PROCÉDÉ ET DISPOSITIF DE TRAITEMENT D'UNE COQUILLE CALCAIRE AU BOUT ARRONDI D'UN OEUF DE POULE

(43) Veröffentlichungstag der Anmeldung: 06.02.2019
(73) Patentinhaber: Agri Advanced Technologies GmbH, 49429 Visbek (DE)
(72) Erfinder: Hurlin, Jörg, 27801 Dötlingen (DE); Meißner, Sven, 09618 Branderbisdorf Stadtteil Oberreichenbach (DE); Fischer, Björn, 09212 Limbach-Oberfrohna OT Wolkenburg (DE)
(74) Vertreter: Vossius & Partner Patentanwälte Rechtsanwälte mbB

(56) Entgegenhaltungen:
- WO-A1-2008/088382
- WO-A1-2015/058076
- WO-A1-2016/019943
- WO-A1-2017/017277
- DE-B3-102016 004 051

## Beschreibung

Die Erfindung betrifft ein Verfahren und eine Vorrichtung zum Bearbeiten einer Kalkschale im Bereich des stumpfen Pols eines Vogeleis. Die Erfindung betrifft insbesondere derartige Verfahren und Vorrichtungen, die die Einbringung einer Schwächungslinie in die Kalkschale, beispielsweise für eine Geschlechtsbestimmung eines Embryos unter Verwendung von Messergebnissen, die nach dem Öffnen des Vogeleis entlang der Schwächungslinie gewonnen werden, ermöglichen.

In der industriellen Geflügelproduktion werden Bruteier von Legehennenlinien oder Geflügelmastlinien zu Beginn des Brutprozesses in sogenannten Bruthorden eingelegt, wobei die Vogeleier derart platziert werden, dass der stumpfe Pol des Vogeleis und somit die im Vogelei befindliche Luftkammer nach oben gerichtet sind. Der etwas spitzere Pol des ovalen Vogeleis ist dabei nach unten gerichtet. Anschließend können die Bruthorden zunächst in einen Hordenwagen oder in Hordenregalen in einen Brutschrank eingebracht werden. Nach einer zeitlich vorgegebenen Vorbrut werden die bebrüteten Vogeleier dann auf sogenannte Schlupfhorden zum Schlüpfen der Küken umgelagert.

Aufgrund der starken Spezialisierung in der Hühnerzucht ist die Geschlechtsbestimmung des sich entwickelnden Kükens von außerordentlicher Bedeutung. Diese erfolgt derzeit in der Praxis häufig unmittelbar nach dem Schlupf manuell oder visuell anhand der Kloakenmorphologie, der Federfarbe oder der Federform bestimmter Federpartien. Die identifizierten Küken werden separiert und in die jeweiligen Vermehrer- oder Produktionsbetriebe gebracht. Insbesondere werden männliche Küken der Legehennenlinien nur in geringer Anzahl zur Zucht benötigt (Eltern-/Großelterntiere) oder sind aufgrund der Genetik nicht für die Mast geeignet und werden direkt nach dem Schlupf aussortiert und getötet.

Das Töten männlicher Eintagsküken stößt zunehmend auf ethische Bedenken.

In herkömmlichen Vorrichtungen zur Geschlechtsbestimmung von bebrüteten Vogeleiern, wie sie beispielsweise in der WO 2011/088825 A1 oder der DE 10 2007 013 107 A1 offenbart sind, wird bei der Bearbeitung einer Kalkschale eine Lochausbildung in der Kalkschale außerhalb des Bereiches der Luftkammer mit Öffnung der beiden Membranen vorgenommen, wobei eine starke Beeinflussung des Bruteies in Kauf genommen wird. Es können Verletzungen auftreten, sodass nach der Geschlechtsbestimmung häufig keine weitere Entwicklung des Embryos möglich ist, sodass der Schlupferfolg deutlich herabgesetzt wird.

Die WO 2017/017277 A1 offenbart Verfahren und Vorrichtungen zum Öffnen einer Kalkschale von Vogeleiern. Diese Verfahren und Vorrichtungen erlauben, dass die innere Membran intakt bleibt. Bei diesen Verfahren und Vorrichtungen wird eine Lage der Luftkammer durch Durchstrahlen des Vogeleis und Detektieren des durch das Vogelei hindurchgetretenen Lichts ermittelt. Derartige Methoden können technisch komplex sein. Darüber hinaus kann eine robuste Bestimmung der Lage der Luftkammer durch verschiedene Farben von Kalkschalen der Vogeleier und/oder auf der Kalkschale vorhandene pigmentartige Flecken erschwert werden. Die robuste Bestimmung der Lage der Luftkammer kann auch erfordern, dass das Umgebungslicht sowie Streulichteinflüsse schwach sind. Es wäre wünschenswert, die Position der Luftblase durch eine alternative robuste, zuverlässige und einfach handhabbare Methode bestimmen zu können.

Die WO 2008/088382 A1 offenbart Verfahren zur Detektion von Schiereiern unter Verwendung von Wärmebildkameras. Die mit der Wärmebildkamera erfassten Daten werden beispielsweise ausgewertet, um invertierte Eier von nicht invertierten Eiern zu unterschieden.

Der Erfindung liegt die Aufgabe zugrunde, ein verbessertes Verfahren und eine verbesserte Vorrichtung zum Bearbeiten der Kalkschale im Bereich des stumpfen Pols von bebrüteten Vogeleiern mit einem darin enthaltenen Embryo anzugeben. Der Erfindung liegt insbesondere die Aufgabe zugrunde, ein Verfahren und eine Vorrichtung anzugeben, mit denen die Position der Luftkammer zuverlässig und in einfacher Weise bestimmbar ist, um die Kalkschale gezielt im Bereich der Luftkammer bearbeiten zu können, wobei die Bearbeitung auch bei Kalkschalen unterschiedlicher Farbe und/oder bei Kalkschalen mit pigmentartigen Flecken eine zuverlässige und robuste Bestimmung der Lage der Luftkammer ermöglichen soll. Dabei sollte in vorteilhafter Weise die Bearbeitung ein Öffnen der Kalkschale derart erlauben, dass das Vogelei nur möglichst schwach in seiner Entwicklung beeinflusst wird.

Diese Aufgaben werden durch ein Verfahren und eine Vorrichtung mit den in den unabhängigen Patentansprüchen angegebenen Merkmalen gelöst. Die abhängigen Patentansprüche definieren Ausführungsformen.

Bei erfindungsgemäßen Vorrichtungen und Verfahren wird eine Lage einer Luftkammer des Vogeleis durch Auswertung einer Abbildung des Vogeleis bestimmt, wobei die Abbildung unter Verwendung einer Wärmebildkamera erfasst wird, deren Ausgangssignal oder Ausgangsdaten im Wesentlichen unabhängig von einer Farbe der Kalkschale ist. Das Verwenden einer Magnetresonanzbildgebungseinrichtung, eines Ultraschallsensors, einer Elektrische Impedanz-Tomographie (EIT)-Bildgebungseinrichtung oder einer anderen Sensoreinrichtung wird hier ebenfalls offenbart, allerdings nicht beansprucht. Die Sensoreinrichtung kann für die Wellenlängen im sichtbaren Bereich des elektromagnetischen Spektrums im Wesentlichen unempfindlich sein. Die Lage der Luftkammer kann durch erfindungsgemäße Vorrichtungen und Verfahren zuverlässig auch dann ermittelt werden, wenn die Kalkschale beispielsweise eine dunkle Farbe oder Farbverläufe aufweist oder wenn stärkere Umgebungslichteinflüsse im sichtbaren Spektralbereich vorhanden sind.

Ein erfindungsgemäßes Verfahren zum Bearbeiten einer Kalkschale eines Vogeleis mit darin enthaltenem Embryo im Bereich des stumpfen Pols des Vogeleis weist die folgenden Schritte auf: Ermitteln einer Lage einer Luftkammer, die zwischen einer inneren Membran und einer äußeren Membran des Vogeleis im Bereich des stumpfen Pols angeordnet ist, und Ansteuern einer Bearbeitungseinrichtung zum Bearbeiten der Kalkschale im Bereich des stumpfen Pols abhängig von der durch das Auswerten der Abbildung ermittelten Lage der Luftkammer. Das Ermitteln der Lage der Luftkammer weist die Schritte auf: Erfassen einer Abbildung des Vogeleis unter Verwendung einer Sensoreinrichtung, deren Ausgangssignal oder Ausgangsdaten von einer Farbe der Kalkschale im Wesentlichen unabhängig sind, und Auswerten der Abbildung zum Ermitteln der Lage der Luftkammer Durch die Verwendung einer Sensoreinrichtung, deren Ausgangssignal oder Ausgangsdaten im Wesentlichen unabhängig von der Farbe der Kalkschale sind, kann die Lage der Luftkammer, insbesondere die Lage eines Zentrums der Luftkammer, am stumpfen Pol des Vogeleis zuverlässig, robust und einfach bestimmt werden. Durch die Ansteuerung der Bearbeitungseinrichtung in Abhängigkeit von der durch das Auswerten der Abbildung ermittelten Lage der Luftkammer kann reproduzierbar und zuverlässig die Kalkschale so bearbeitet werden, dass das Vogelei beispielsweise ohne Beschädigung der inneren Membran am stumpfen Pol geöffnet werden kann.

Unter der Bestimmung der Lage einer Luftkammer wird die Bestimmung eines Bereichs der Kalkschale, an den die Luftkammer an der Innenseite der Kalkschale angrenzt, und/oder die Bestimmung eines Volumens im Inneren des Vogeleis, der von der Luftkammer eingenommen ist, verstanden. Die Bestimmung der Lage der Luftkammer kann die Bestimmung eines geometrischen Mittelpunkts des Bereichs der Kalkschale, an den die Luftkammer an der Innenseite der Kalkschale angrenzt, aufweisen. Die Bestimmung der Lage der Luftkammer kann optional die Bestimmung einer oder mehrerer Abmessungen des Bereichs der Kalkschale, an den die Luftkammer an der Innenseite der Kalkschale angrenzt, aufweisen.

Die Sensoreinrichtung weist eine Wärmebildkamera auf. Die zur Ermittlung der Lage der Luftkammer ausgewertete Abbildung kann ein Wärmebild sein. Das Wärmebild kann mit einer Wärmebildkamera aufgenommen werden, deren Spektralbereich das Wellenlängenintervall von 8 µm bis 14 µm aufweist. Das Wärmebild kann mit einer Wärmebildkamera aufgenommen werden, deren Temperaturauflösung bei 30°C und einer Aufnahmerate von 50 Hz kleiner oder gleich 80 mK ist.

Die Sensoreinrichtung kann zusätzlich zur Wärmebildkamera eine Magnetresonanzbildgebungseinrichtung aufweisen.

Die Sensoreinrichtung kann zusätzlich zur Wärmebildkamera einen Ultraschallsensor aufweisen.

Die Sensoreinrichtung kann zusätzlich zur Wärmebildkamera eine Elektrische Impedanz-Tomographie (EIT)-Bildgebungseinrichtung aufweisen.

Die Sensoreinrichtung kann konfiguriert sein, elektromagnetische Wellenlängen zu erfassen, die größer als 780 nm sind. Die Sensoreinrichtung kann einen Infrarotsensor umfassen.

Die Sensoreinrichtung kann konfiguriert sein, akustische Wellen mit einer Frequenz größer als 16 kHz zu erfassen.

Das Auswerten der Abbildung weist eine Segmentierung der Abbildung zum Ermitteln der Lage einer Projektion der Luftkammer in einer Bildebene der Sensoreinrichtung auf, wobei die Segmentierung eine kantenorientierte Segmentierungsmethode oder eine regionenorientierte Segmentierungsmethode aufweist. Die Segmentierung der Abbildung kann eine rechnerische Vorverarbeitung der Abbildung, beispielsweise zur Verstärkung des Kontrasts, aufweisen.

Die Segmentierung kann zusätzlich einen pixel- oder voxelweisen Vergleich der Abbildung mit einem Schwellenwert aufweisen.

Der Schwellenwert kann von einer Dauer einer Zeitspanne zwischen einem Entnehmen des Vogeleis aus einer Einrichtung zum Bebrüten des Vogeleis und dem Erfassen der Abbildung abhängen.

Das Auswerten der Abbildung kann eine Bestimmung eines Flächenmittelpunkts der Projektion der Luftkammer auf die Bildebene der Sensoreinrichtung aufweisen.

Das Auswerten der Abbildung kann eine Bestimmung eines Volumenmittelpunkts der Luftkammer aufweisen.

Durch das Auswerten der Abbildung kann die Lage der Luftkammer, insbesondere die Lage eines Mittelpunkts der Luftkammer, zuverlässig und robust bestimmt werden.

Durch das Auswerten der Abbildung kann zusätzlich auch eine Abmessung der Luftkammer ermittelt werden. Die ermittelte Abmessung der Luftkammer kann eine geschlossene Linie auf der Kalkschale so definieren, dass an der Innenseite der Kalkschale entlang der geschlossenen Linie die Luftkammer angeordnet ist.

Das Verfahren kann ein Bestimmen von Abmessungen des Vogeleis aufweisen. Dazu kann mit wenigstens einer von der Sensoreinrichtung verschiedenen Detektionseinrichtung ein zwei- oder dreidimensionales Bild des Vogeleis aufgenommen werden. Das zwei- oder dreidimensionales Bild kann mit der durch Auswerten der Abbildung ermittelten Lage der Luftkammer kombiniert werden, um eine Linie auf einer Oberfläche der Kalkschale des Vogeleis zu bestimmen, entlang der eine Schwächungslinie eingebracht oder die Kalkschale anderweitig bearbeitet wird. Abhängig von dem zwei- oder dreidimensionalen Bild und abhängig von der durch Auswerten der Abbildung ermittelten Lage der Luftkammer können die Koordinaten von Punkten auf der Kalkschale bestimmt werden, die die Schwächungslinie oder einen Schwächungsbereich definieren.

Die Bearbeitungseinrichtung kann abhängig von der durch das Auswerten der Abbildung ermittelten Lage der Luftkammer so angesteuert werden, dass eine Schwächungslinie erzeugt wird, die in einem Bereich der Kalkschale angeordnet ist, dessen Innenseite an die Luftkammer angrenzt. Die Bearbeitungseinrichtung kann abhängig von der durch das Auswerten der Abbildung ermittelten Lage der Luftkammer so angesteuert werden, dass die gesamte Schwächungslinie einen vorgegebenen Mindestabstand von der inneren Membran aufweist.

Die Bearbeitungseinrichtung kann abhängig von der durch das Auswerten der Abbildung ermittelten Lage der Luftkammer so angesteuert werden, dass eine Richtung, aus der die Bearbeitungseinrichtung auf die Kalkschale einwirkt, von der durch das Auswerten der Abbildung ermittelten Lage der Luftkammer abhängt. Auf diese Weise kann die Kalkschale so bearbeitet werden, dass die Kalkschale zuverlässig ohne negative Beeinflussung des Embryos im Vogelei geöffnet werden kann.

Die Bearbeitungseinrichtung kann konfiguriert sein, eine Schwächungslinie in die Kalkschale einzubringen.

Die Bearbeitungseinrichtung kann eine Quelle elektromagnetischer Strahlung, beispielsweise eine Laserlichtquelle, und eine steuerbare Umlenkeinrichtung aufweisen. Die Umlenkeinrichtung kann abhängig von der durch das Auswerten der Abbildung ermittelten Lage der Luftkammer angesteuert werden. Falls auch ein zwei- oder dreidimensionales Bild des Vogeleis erfasst wird, um die Geometrie des Vogeleis zu bestimmen, kann die Umlenkeinrichtung abhängig von der durch das Auswerten der Abbildung ermittelten Lage der Luftkammer und abhängig von der Geometrie des Vogeleis angesteuert werden.

Da die Sensoreinrichtung eine Wärmebildkamera aufweist, kann Temperaturinformation des Wärmebilds zur Qualitätssicherung und/oder Prozesssteuerung eingesetzt werden. Beispielsweise kann abhängig von aus dem Wärmebild gewonnener Temperaturinformation bestimmt werden, ob das Vogelei in einem für die Brut ungeeigneten Temperaturbereich gehalten wurde. Abhängig von aus dem Wärmebild gewonnener Temperaturinformation kann das Vogelei selektiv aussortiert werden.

Alternativ oder zusätzlich kann Temperaturinformation, die aus dem Wärmebild gewonnen wird, zur Prozessführung wenigstens eines Teils eines Brutprozesses des Vogeleis eingesetzt werden. Beispielsweise kann eine Bruteinrichtung für das Vogelei und/oder eine Transporteinrichtung für den Transport des Vogeleis abhängig von der aus dem Wärmebild gewonnenen Temperaturinformation gesteuert oder geregelt werden.

Ein erfindungsgemäßes Verfahren zur Geschlechtsbestimmung eines in einem bebrüteten Vogelei enthaltenen Embryos anhand einer Messung am stumpfen Pol des Vogeleis weist die folgenden Schritte auf: Einbringen einer Schwächungslinie in eine Kalkschale des Vogeleis mit dem erfindungsgemäßen Verfahren zur Bearbeitung der Kalkschale; Abheben eines durch die Schwächungslinie definierten Bereichs der Kalkschale zum Öffnen des Vogeleis; Ausführen wenigstens einer Messung an dem geöffneten Vogelei zum Bestimmen des Geschlechts des Embryos; und Verschließen der geöffneten Kalkschale des Vogeleis durch Aufsetzen eines Verschlussmaterials.

Da die Schwächungslinie bei dem Verfahren zur Geschlechtsbestimmung abhängig von der durch das Auswerten der Abbildung ermittelten Lage der Luftkammer erfasst wird, kann mit dem Verfahren das Vogelei zuverlässig derart für die Messung zum Bestimmen des Geschlechts des Embryos geöffnet werden, dass das Risiko einer negativen Beeinflussung des Embryos auch bei unterschiedlichen Umgebungsbedingungen oder für Vogeleier mit verschiedener Farbe der Kalkschale klein ist.

Das Abheben des durch die Schwächungslinie definierten Bereichs kann abhängig von einem nach dem Einbringen der Schwächungslinie erfassten Bild des Vogeleis ausgeführt werden. Eine Öffnungseinrichtung kann abhängig von dem erfassten Bild des Vogeleis nach dem Einbringen der Schwächungslinie angesteuert werden. Insbesondere kann eine Richtung, von der aus die Öffnungseinrichtung auf das Vogelei einwirkt, abhängig von der Lage der Schwächungslinie angesteuert werden, die wiederum von der durch das Auswerten der Abbildung ermittelten Lage der Luftkammer abhängt.

Eine erfindungsgemäße Vorrichtung zum Bearbeiten einer Kalkschale eines Vogeleis mit darin enthaltenem Embryo im Bereich des stumpfen Pols des Vogeleis weist auf: eine Sensoreinrichtung, deren Ausgangssignal oder Ausgangsdaten von einer Farbe der Kalkschale im Wesentlichen unabhängig sind, zum Erfassen einer Abbildung des Vogeleis; eine Steuereinrichtung zum Auswerten der Abbildung, um eine Lage einer Luftkammer, die zwischen einer inneren Membran und einer äußeren Membran des Vogeleis im Bereich des stumpfen Pols angeordnet ist, zu bestimmen; und eine Bearbeitungseinrichtung, die von der Steuereinrichtung steuerbar ist, zum Bearbeiten der Kalkschale im Bereich des stumpfen Pols abhängig von der durch die Steuereinrichtung ermittelten Lage der Luftkammer.

Die Bearbeitungseinrichtung kann konfiguriert sein, eine Schwächungslinie in die Kalkschale im Bereich des stumpfen Pols abhängig von der durch Auswerten der Abbildung ermittelten Lage der Luftkammer einzubringen.

Die Steuereinrichtung ist konfiguriert, die Lage einer Projektion der Luftkammer in einer Bildebene der Sensoreinrichtung durch Segmentierung der Abbildung unter Verwendung einer kantenorientierten Segmentierungsmethode oder einer regionenorientierten Segmentierungsmethode zu bestimmen.

Die Steuereinrichtung kann zusätzlich konfiguriert sein, zur Segmentierung der Abbildung einen pixel- oder voxelweisen Vergleich der Abbildung mit einem Schwellenwert auszuführen.

Die Steuereinrichtung kann derart konfiguriert sein, dass der Schwellenwert von der Dauer der Zeitspanne zwischen einem Entnehmen des Vogeleis aus einer Einrichtung zum Bebrüten des Vogeleis und dem Erfassen der Abbildung abhängt.

Die Steuereinrichtung kann konfiguriert sein, einen Flächenmittelpunkt der Projektion der Luftkammer auf die Bildebene der Sensoreinrichtung zu ermitteln.

Die Sensoreinrichtung weist eine Wärmebildkamera zum Erfassen eines Wärmebilds auf.

Ein Spektralbereich der Wärmebildkamera kann das Wellenlängenintervall von 8 µm bis 14 µm aufweisen. Eine Temperaturauflösung der Wärmebildkamera bei 30°C und einer Aufnahmerate von 50 Hz kann kleiner oder gleich 80 mK sein.

Die Sensoreinrichtung kann zusätzlich zur Wärmebildkamera eine Magnetresonanzbildgebungseinrichtung zum Erfassen eines Magnetresonanzbilds aufweisen.

Die Sensoreinrichtung kann zusätzlich zur Wärmebildkamera einen Ultraschallsensor zum Erfassen eines Ultraschallbilds aufweisen.

Die Sensoreinrichtung kann zusätzlich zur Wärmebildkamera eine EIT-Bilderfassungseinrichtung zum Erfassen eines EIT-Bilds aufweisen.

Die Sensoreinrichtung kann konfiguriert sein, elektromagnetische Wellenlängen größer als 780 nm zu erfassen.

Die Sensoreinrichtung kann konfiguriert sein, akustische Wellen mit einer Frequenz größer als 16 kHz zu erfassen.

Die Vorrichtung kann wenigstens eine von der Sensoreinrichtung verschiedene Detektionseinrichtung aufweisen, die konfiguriert ist, ein zwei- oder dreidimensionales Bild des Vogeleis aufzunehmen. Die Steuereinrichtung kann konfiguriert sein, das zwei- oder dreidimensionales Bild mit der durch Auswerten der Abbildung ermittelten Lage der Luftkammer zu kombinieren, um eine Linie auf einer Oberfläche der Kalkschale des Vogeleis zu bestimmen, entlang der das Vogelei bearbeitet wird. Die Steuereinrichtung kann konfiguriert sein, abhängig von dem zwei- oder dreidimensionalen Bild und der durch Auswerten der Abbildung ermittelten Lage der Luftkammer die Koordinaten von Punkten auf der Kalkschale zu bestimmen, die die Schwächungslinie definieren.

Die Steuereinrichtung kann konfiguriert sein, die Bearbeitungseinrichtung abhängig von der durch das Auswerten der Abbildung ermittelten Lage der Luftkammer so anzusteuern, dass die gesamte Schwächungslinie in einem Bereich der Kalkschale angeordnet ist, dessen Innenseite die Luftkammer begrenzt. Die Steuereinrichtung kann konfiguriert sein, die Bearbeitungseinrichtung abhängig von der durch das Auswerten der Abbildung ermittelten Lage der Luftkammer so anzusteuern, dass die gesamte Schwächungslinie einen vorgegebenen Mindestabstand von der inneren Membran aufweist.

Die Bearbeitungseinrichtung kann derart konfiguriert sein, dass eine Richtung, aus der die Bearbeitungseinrichtung auf die Kalkschale einwirkt, von der durch das Auswerten der Abbildung ermittelten Lage der Luftkammer abhängt.

Die Vorrichtung kann eine Öffnungseinrichtung zum Öffnen eines durch die Bearbeitungseinrichtung bearbeiteten Bereichs der Kalkschale aufweisen. Die Öffnungseinrichtung kann konfiguriert sein, die Kalkschale zu öffnen, ohne die innere Membran zu beschädigen.

Die Vorrichtung kann eine Messeinrichtung zum Ausführen wenigstens einer Messung an dem geöffneten Vogelei zum Bestimmen eines Geschlechts des Embryos aufweisen.

Die Vorrichtung kann eine Verschließeinrichtung zum Verschließen der geöffneten Kalkschale des Vogeleis aufweisen.

Bei den Vorrichtungen und Verfahren kann die Messung zur Bestimmen des Geschlechts des Embryos die Erfassung eines Raman-Spektrums, eines Fluoreszenzsspektrums und/oder eines Absorptionsspektrums aufweisen. Bei den Vorrichtungen und Verfahren kann eine IR-Raman-Spektroskopie ausgeführt und das erfasste Raman-Spektrum oder mehrere erfasste Raman-Spektren ausgewertet werden, um das Geschlecht des Embryos zu bestimmen.

Nach einem weiteren Aspekt der Erfindung wird ein Verfahren zum Einbringen einer Schwächungslinie in eine Kalkschale eines Vogeleis mit darin enthaltenem Embryo im Bereich des stumpfen Pols des Vogeleis angegeben, das die folgenden Schritte aufweist: Erfassen eines Wärmebilds des Vogeleis, Auswerten des Wärmebilds zum Bestimmen einer Lage einer Luftkammer, die zwischen einer inneren Membran und einer äußeren Membran des Vogeleis im Bereich des stumpfen Pols angeordnet ist, und Ansteuern einer Bearbeitungseinrichtung zum Einbringen einer Schwächungslinie in die Kalkschale im Bereich des stumpfen Pols abhängig von der durch das Auswerten des Wärmebilds ermittelten Lage der Luftkammer.

Nach einem weiteren Aspekt der Erfindung wird ein Verfahren zur Geschlechtsbestimmung eines in einem bebrüteten Vogelei enthaltenen Embryos anhand einer Messung am stumpfen Pol des Vogeleis angegeben, das die folgenden Schritte aufweist: Einbringen einer Schwächungslinie in eine Kalkschale des Vogeleis mit dem im vorhergehenden Absatz beschriebenen Verfahren, Abheben eines durch die Schwächungslinie definierten Bereichs der Kalkschale zum Öffnen des Vogeleis, Ausführen wenigstens einer Messung an dem geöffneten Vogelei zum Bestimmen des Geschlechts des Embryos und Verschließen der geöffneten Kalkschale des Vogeleis durch Aufsetzen eines Verschlussmaterials.

Nach einem weiteren Aspekt der Erfindung wird eine Vorrichtung zum Einbringen einer Schwächungslinie in eine Kalkschale eines Vogeleis mit darin enthaltenem Embryo im Bereich des stumpfen Pols des Vogeleis angegeben. Die Vorrichtung weist auf: eine Wärmebildkamera zum Erfassen eines Wärmebilds des Vogeleis, eine Steuereinrichtung zum Auswerten des Wärmebilds zum Bestimmen einer Lage einer Luftkammer, die zwischen einer inneren Membran und einer äußeren Membran des Vogeleis im Bereich des stumpfen Pols angeordnet ist, und eine Bearbeitungseinrichtung, die von der Steuereinrichtung steuerbar ist, zum Einbringen einer Schwächungslinie in die Kalkschale im Bereich des stumpfen Pols abhängig von der durch die Steuereinrichtung durch Auswerten des Wärmebilds ermittelten Lage der Luftkammer.

Diese Verfahren und Vorrichtungen können nach Ausführungsbeispielen die vorangehend bereits ausführlich beschriebenen optionalen Merkmale aufweisen.

Mit den Vorrichtungen und Verfahren kann eine Schwächungslinie durch Verwendung von Laserstrahlung in einem Bereich des stumpfen Pols des Vogeleis eingebracht werden. Die Schwächungslinie kann eine Sollbruchlinie der Kalkschale definieren. Die Schwächungslinie kann als grabenförmige Sollbruchlinie in den Bereich des stumpfen Pols des Vogeleis derart eingebracht werden, dass bei Entfernung des innerhalb der Sollbruchlinie liegenden Teils der Kalkschale die Luftkammer freigelegt wird, ohne dass die innere Membran des Vogeleis beschädigt wird.

Die Verfahren und Vorrichtungen nach Ausführungsbeispielen erlauben eine Bestimmung der Lage der Luftkammer, um die Kalkschale gezielt im Bereich der Luftkammer des Vogeleis zu bearbeiten. Die Bestimmung der Lage der Luftkammer kann auch für Kalkschalen unterschiedlicher Farben und bei Änderungen der Lichtverhältnisse einfach und zuverlässig erfolgen. Die Geschlechtsbestimmung des Embryos kann auf eine Weise durchgeführt werden, die das Brutei nicht oder nur geringfügig in seiner Entwicklung beeinträchtigt.

Ausführungsbeispiele der Erfindung werden unter Bezugnahme auf die Figuren detailliert beschrieben, in denen identische Bezugszeichen identische Elemente bezeichnen.
Figur 1 zeigt eine schematische Draufsicht einer Vorrichtung nach einem Ausführungsbeispiel.
Figur 2 zeigt eine schematische Seitenansicht einer Vorrichtung nach einem Ausführungsbeispiel.
Figur 3 und Figur 4 sind schematische Darstellungen zur Erläuterung der Funktionsweise einer Vorrichtung nach einem Ausführungsbeispiel.
Figur 5 veranschaulicht eine mit der Sensoreinrichtung der Vorrichtung erfasste Abbildung mehrerer Vogeleier.
Figur 6 veranschaulicht eine anhand der Abbildung der mehreren Vogeleier vorgenommene Segmentierung zur Bestimmung einer Lage einer Luftkammer.
Figur 7 ist ein Flussdiagramm eines Verfahrens nach einem Ausführungsbeispiel.
Figur 8 bis Figur 17 zeigen Ergebnisse einer Segmentierung von Wärmebildern von Vogeleiern, die an denselben Vogeleiern zu unterschiedlichen Zeiten nach einer Entnahme aus einer Bruteinrichtung aufgenommen wurden.
Figur 18 zeigt die anhand der in Figur 8 bis Figur 17 durchgeführten Segmentierung bestimmten Mittelpunkte der Projektion der Luftkammer in einer Bildebene einer Wärmebildkamera.
Figur 19 ist eine grafische Darstellung einer Zeitabhängigkeit der Temperatur der in Wärmebildern insgesamt sichtbaren Kalkschalenoberfläche und einer Zeitabhängigkeit der Temperatur eines die Luftkammer überdeckenden Bereichs der Kalkschalenoberfläche.
Figur 20 zeigt eine Seitenansicht einer Vorrichtung nach einem Ausführungsbeispiel.
Figur 21 ist eine schematische Darstellung zur Erläuterung der Funktionsweise einer Vorrichtung nach einem Ausführungsbeispiel.
Figur 22 ist eine schematische Darstellung zur Erläuterung der Funktionsweise einer Vorrichtung nach einem Ausführungsbeispiel.
Figur 23 ist ein Flussdiagramm eines Verfahrens nach einem Ausführungsbeispiel.

Nachfolgend werden bevorzugte und vorteilhafte Ausführungsbeispiele der Erfindung unter Bezugnahme auf die Figuren beschrieben, in denen identische Bezugszeichen identische oder ähnliche Elemente bezeichnen. Die Merkmale der verschiedenen Ausführungsbeispiele können miteinander kombiniert werden, sofern dies in der nachfolgenden Beschreibung nicht ausdrücklich ausgeschlossen wird.

Nach Ausführungsbeispielen der Erfindung wird für die Bestimmung einer Lage einer Luftkammer des Vogeleis eine Sensoreinrichtung verwendet, die von einer Farbbildkamera verschieden ist. Die Sensoreinrichtung kann eine Wärmebildkamera, eine Magnetresonanzbildgebungseinrichtung, ein Ultraschallsensor oder eine Elektrische Impedanz-Tomographie (EIT)-Bildgebungseinrichtung aufweisen.

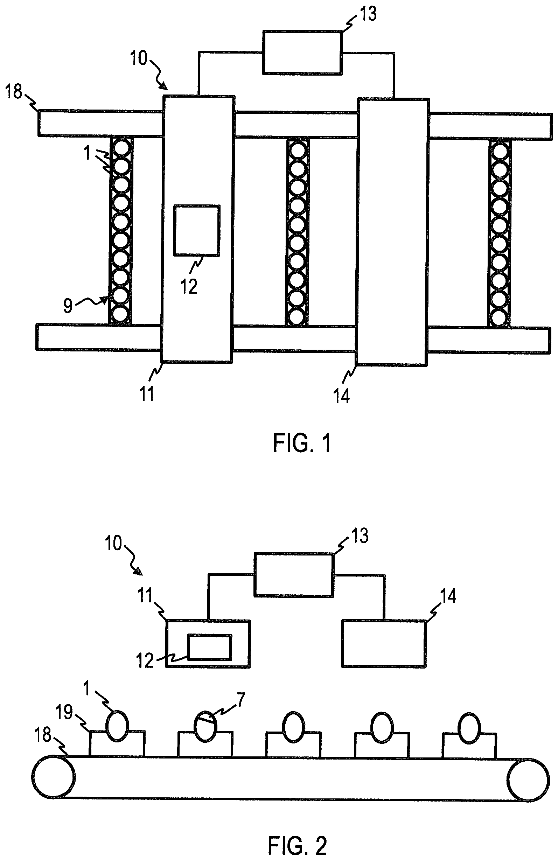

Figuren 1 und 2 zeigen schematische Ansichten einer Vorrichtung 10 zum Bearbeiten einer Kalkschale eines Vogeleis 1. Die Vorrichtung 10 kann zum Einbringen einer Schwächungslinie in die Kalkschale des Vogeleis 1 mit darin enthaltenem Embryo im Bereich des stumpfen Pols des Vogeleis konfiguriert sein. Die Vorrichtung 10 kann eingesetzt werden, um die Kalkschale zum gezielten Öffnen des Vogeleis am stumpfen Pol im Bereich der Luftkammer 7 für eine Geschlechtsbestimmung des Embryos zu bearbeiten.

Die Vorrichtung 10 weist eine Sensoreinrichtung 11 auf. Die Sensoreinrichtung 11 weist eine Wärmebildkamera 12 zum Erfassen eines Wärmebilds des Vogeleis auf. Die Wärmebildkamera 12 kann beispielsweise in einem Spektralbereich sensitiv sein, der das Wellenlängenintervall von 8 µm bis 14 µm beinhaltet. Die Wärmebildkamera kann bei einer Temperatur von 30°C und einer Aufnahmerate von 50 Hz eine Temperaturauflösung aufweisen, die kleiner oder gleich 80 mK sein kann. Andere Wärmebildkameras 12 können eingesetzt werden.

Zusätzlich zur Wärmebildkamera 12 kann die Sensoreinrichtung 11 eine Magnetresonanzbildgebungseinrichtung, einen Ultraschallsensor oder eine EIT-Bildgebungseinrichtung aufweisen, um eine Abbildung für die Ermittlung der Lage der Luftkammer 7 zu erfassen.

Die Sensoreinrichtung 11 kann angeordnet sein, eine Abbildung eines einzelnen Vogeleis oder eine Abbildung mehrerer Vogeleier 9 gleichzeitig aufzunehmen. Die Vogeleier 1 können beispielsweise in Halterungen 19 einzeln oder in Bruthorden angeordnet sein. Die Sensoreinrichtung 11 kann gleichzeitig eine Abbildung mehrerer Vogeleier erfassen, die anschließend von einer Steuereinrichtung 13 ausgewertet wird. Die Steuereinrichtung 13 kann die Abbildung auswerten, um die Lage der Luftkammer 7 des Vogeleis 1 oder die Lage der Luftkammern 7 mehrerer Vogeleier zu bestimmen.

Die Vorrichtung 10 weist eine steuerbare Bearbeitungseinrichtung 14 auf. Die Bearbeitungseinrichtung 14 ist konfiguriert, die Kalkschale des Vogeleis 1 zu bearbeiten. Die Bearbeitungseinrichtung 14 kann konfiguriert sein, die Kalkschale abhängig von der ermittelten Lage der Luftkammer so zu bearbeiten, dass die Kalkschale im Bereich der Luftkammer geschwächt, aber nicht vollständig durchtrennt wird. Die Bearbeitungseinrichtung 14 kann konfiguriert sein, eine grabenförmige Schwächungslinie in die Kalkschale des Vogeleis 1 einzubringen, die eine Vertiefung mit einem Boden in der Kalkschale bildet, ohne die Kalkschale an irgendeinem Punkt vollständig zu durchtrennen. Ein Aufbrechen der Kalkschale entlang der Schwächungslinie kann durch eine in Prozessrichtung nachgeordnete Öffnungseinrichtung vorgenommen werden.

Die steuerbare Bearbeitungseinrichtung 14 kann konfiguriert sein, aus unterschiedlichen Richtungen auf das Vogelei 1 einzuwirken, um die Schwächungslinie in Abhängigkeit von der durch Auswertung der Abbildung ermittelten Lage der Luftkammer 7 in die Kalkschale einzubringen. Die steuerbare Bearbeitungseinrichtung 14 kann konfiguriert sein, die Schwächungslinie so in die Kalkschale des Vogeleis 1 einzubringen, dass an jedem Punkt entlang der Schwächungslinie die Innenseite der Kalkschale an die Luftkammer angrenzt.

Die steuerbare Bearbeitungseinrichtung 14 kann eine Quelle elektromagnetischer Strahlung, insbesondere eine Laserlichtquelle, aufweisen. Die Bearbeitungseinrichtung 14 kann eine Umlenkeinrichtung zum Umlenken der elektromagnetischen Strahlung, insbesondere zum Ablenken von Laserlicht, aufweisen. Die Umlenkeinrichtung kann abhängig von der durch Auswertung der Abbildung ermittelten Lage der Luftkammer 7 gesteuert werden, um die Schwächungslinie so in die Kalkschale einzubringen, dass sie ein schonendes Öffnen des Vogeleis 1 im Bereich der Luftkammer 7 am stumpfen Pol ermöglicht. Die Umlenkeinrichtung kann ein reflektierendes Element, insbesondere einen Spiegel, und einen Aktuator zum Verstellen des reflektierenden Elements in Abhängigkeit von der durch Auswertung der Abbildung ermittelten Lage der Luftkammer 7 aufweisen.

Die Quelle elektromagnetischer Strahlung der Bearbeitungseinrichtung 14 kann einen CO₂-Laser, einen Nd:YAG-Laser, einen Er:YAG-Laser oder einen anderen Laser zur Bearbeitung der Kalkschale von Vogeleiern aufweisen.

Die Vorrichtung 10 weist eine Steuereinrichtung 13 auf, die konfiguriert ist, die Abbildung des Vogeleis 1 zum Bestimmen der Lage der Luftkammer 7 im Bereich des stumpfen Pols des Vogeleis 1 auszuwerten und um die Bearbeitungseinrichtung 14 gemäß der durch Auswertung der Abbildung bestimmten Lage der Luftkammer 7 so anzusteuern, dass die Kalkschale im Bereich der Luftkammer 7 und in einem Abstand von der inneren Membran des Vogeleis 1 bearbeitet wird.

Die Steuereinrichtung 13 kann eine oder mehrere integrierte Halbleiterschaltungen aufweisen. Die Steuereinrichtung 13 kann eine oder mehrere von einer anwendungsspezifischen Spezialschaltung, einem Controller, einem Mikrocontroller, einem Prozessor, einem Mikroprozessor oder Kombinationen davon aufweisen.

Die Steuereinrichtung 13 kann konfiguriert sein, eine Segmentierung der mit der Sensoreinrichtung 11 erfassten Abbildung vorzunehmen, um die Lage einer Projektion der Luftkammer in einer Bildebene der Sensoreinrichtung 11 zu bestimmen. Die Segmentierung kann einen bildpunktabhängigen Vergleich der Abbildung mit einem Schwellenwert aufweisen. Erfindungsgemäß werden kantenorientierte Segmentierungsmethoden, beispielsweise ein Kantenverfolgungsalgorithmus, eine Wasserscheidentransformation, eine parallele oder sequentielle Kantenextraktion oder andere kantenorientierte Segmentierungsmethoden, oder regionenorientierte Segmentierungsmethoden, wie beispielsweise Energiemethoden, Region Growing, Region-Splitting, Pyramid Linking und Split and Merge, auf die Abbildung angewandt.

Durch die Segmentierung kann die Steuereinrichtung 13 einen zusammenhängenden Bereich in der Abbildung des Vogeleis identifizieren, der der Luftkammer entspricht. Falls beispielsweise die Sensoreinrichtung 11 eine Wärmebildkamera 12 aufweist, kann die Steuereinrichtung 13 einen zusammenhängenden Bereich des Wärmebilds, dessen Temperatur kleiner als die Temperatur eines ihn umgebenden Bereich ist, als die Projektion der Luftkammer in der Bildebene der Sensoreinrichtung 11 identifizieren.

Punkte in dem durch Segmentierung ermittelten Bereich der Abbildung, der der Luftkammer entspricht, können eine Temperatur aufweisen, die kleiner als ein Schwellenwert ist. Punkte außerhalb dieses Bereichs können jedenfalls im Mittel eine Temperatur aufweisen, die größer als der Schwellenwert ist. Der Schwellenwert kann von einer Dauer einer Zeitspanne zwischen einem Entnehmen des Vogeleis aus einer Einrichtung zum Bebrüten des Vogeleis 1 und dem Erfassen der Abbildung abhängen. Die Steuereinrichtung 13 kann konfiguriert sein, die Dauer der Zeitspanne zwischen dem Entnehmen des Vogeleis aus der Einrichtung zum Bebrüten des Vogeleis 1 und dem Erfassen der Abbildung bei der Segmentierung als Parameter zu verwenden. Beispielsweise kann der Schwellenwert in Abhängigkeit von der Dauer der Zeitspanne in einer Kalibrierung ermittelt und nicht-flüchtig gespeichert werden. Der Schwellenwert für die Temperatur muss bei der Segmentierung der Abbildung durch die Steuereinrichtung 13 nicht zwingend explizit bekannt sein, sondern kann beispielsweise bei kanten- oder regionenorientierte Segmentierungsmethoden implizit als Ergebnis der Segmentierung zur Verfügung stehen.

Die Identifizierung der Projektion der Luftkammer in der Bildebene der Sensoreinrichtung 11 durch Segmentierung der Abbildung kann in ähnlicher Weise ebenfalls vorgenommen werden, falls die Abbildung des Vogeleis 1 nicht ein Wärmebild, sondern ein Magnetresonanzbild, ein Ultraschallbild oder ein EIT-Bild ist.

Die Steuereinrichtung 13 kann eine Vorverarbeitung der Abbildung vor der Segmentierung ausführen. Beispielsweise können Filterungsoperationen und/oder Glättungsoperationen auf die Abbildung angewandt werden. Die Segmentierung kann an der gefilterten und/oder geglätteten Abbildung ausgeführt werden.

Die Steuereinrichtung 13 kann konfiguriert sein, einen Flächenmittelpunkt der Projektion der Luftkammer in der Bildebene der Sensoreinrichtung 11 zu bestimmen. Dazu kann die Steuereinrichtung 13 beispielsweise den Flächenmittelpunkt des durch Segmentierung bestimmten Bereichs der Abbildung, der der Luftkammer entspricht, berechnen. Alternativ oder zusätzlich kann die Steuereinrichtung 13 konfiguriert sein, den durch Segmentierung bestimmten Bereichs der Abbildung, der der Luftkammer entspricht, durch eine Ellipse zu approximieren, wobei der Flächenmittelpunkt durch den Schnittpunkt der Halbachsen der Ellipse bestimmt wird.

Die Steuereinrichtung 13 kann zur Durchführung weiterer Steuer- und Auswertefunktionen konfiguriert sein. Beispielsweise kann die Steuereinrichtung 13 konfiguriert sein, die Temperaturinformation eines von der Wärmebildkamera 12 erfassten Wärmebilds für eine Qualitätssicherung zu verwenden. Die Steuereinrichtung 13 kann konfiguriert sein, abhängig von der Temperaturinformation des Wärmebilds zu ermitteln, ob das Vogelei eine für die Brut ungünstige Temperatur aufweist und/oder für eine zu lange Zeitspanne aus der Einrichtung zum Bebrüten des Vogeleis entnommen ist. Abhängig von der Temperaturinformation des Wärmebilds kann die Steuereinrichtung 13 eine automatische Aussortierung von Vogeleiern, für die das Wärmebild eine nicht akzeptable Temperaturführung des bisherigen Brutprozesses anzeigt, und/oder die Ausgabe von Warninformation über eine Benutzerschnittstelle veranlassen.

Die Vorrichtung 10 kann eine Transporteinrichtung 18 aufweisen. Die Transporteinrichtung 18 kann einen Endlosförderer aufweisen. Die Transporteinrichtung 18 kann konfiguriert sein, Vogeleier 1 einzeln oder in einer Gruppe 9 zu der Sensoreinrichtung 11 und anschließend zur Bearbeitungseinrichtung 14 zu fördern. Optional können Vogeleier 1 abhängig von der Auswertung der Abbildung, die von der Sensoreinrichtung 11 erfasst wird, sortiert werden. Die Vogeleier 1 können an dem Förderer 18 in Halterungen 19 gehalten werden.

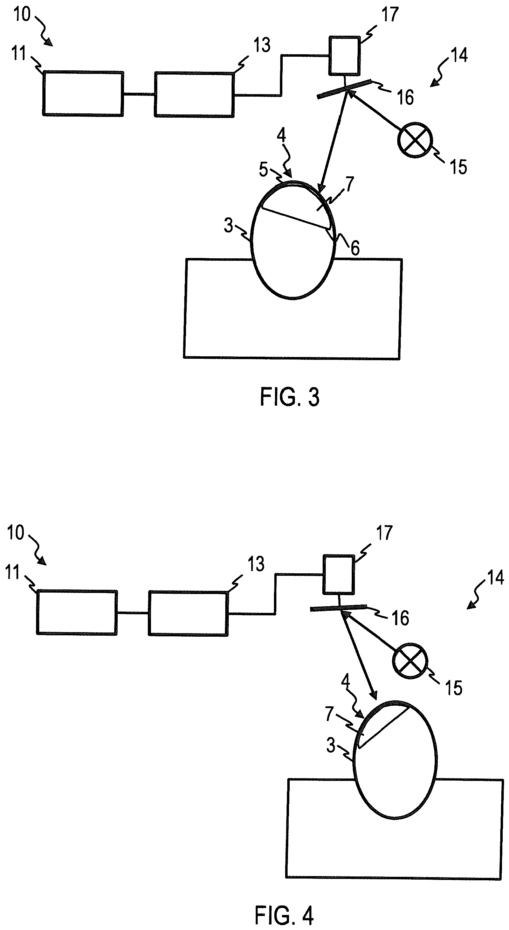

Figur 3 und Figur 4 zeigen schematische Ansichten einer Vorrichtung 10 nach einem Ausführungsbeispiel zur näheren Erläuterung der Funktionsweise einer Implementierung der Bearbeitungseinrichtung 14.

Wie in Figur 3 und Figur 4 dargestellt, kann die Bearbeitungseinrichtung 14 eingerichtet sein, von verschiedenen Richtungen aus auf eine Kalkschale 3 des Vogeleis 1 einzuwirken. Abhängig von der durch Auswertung eines Wärmebilds, eines Magnetresonanzbilds, eines Ultraschallbilds oder eines EIT-Bilds ermittelten Lage der Luftkammer 7 kann die Bearbeitungseinrichtung 14 und optional die Transporteinrichtung 18 von der Steuereinrichtung 13 so angesteuert werden, dass die Bearbeitungseinrichtung 14 in einer Richtung auf die Kalkschale 3 einwirkt, die spezifisch die Bearbeitung im die Luftkammer 7 überdeckenden Bereich der Kalkschale 3 und in der Nähe des stumpfen Pols 4 ermöglicht. Die Luftkammer 7 ist zwischen der inneren Membran 6 und der äußeren Membran 5 des Vogeleis angeordnet. Die Steuereinrichtung 13 kann die Bearbeitungseinrichtung 14 so ansteuern, dass die Bearbeitungseinrichtung 14 den unmittelbar die Luftkammer 7 überdeckenden Bereich der Kalkschale 3 bearbeitet, ohne die innere Membran 6 zu verletzen.

Die Bearbeitungseinrichtung 14 kann eine Quelle 15 elektromagnetischer Strahlung aufweisen. Die Quelle 15 kann einen Laser aufweisen. Die Quelle 15 kann einen CO₂-Laser, einen Nd:YAG-Laser, einen Er:YAG-Laser oder einen anderen Laser zur Bearbeitung der Kalkschale 3 aufweisen. Die Bearbeitungseinrichtung 14 kann eine Umlenkeinrichtung 16 für die von der Quelle 15 ausgegebene elektromagnetische Strahlung, beispielsweise einen Laserstrahl, aufweisen. Die Umlenkeinrichtung 16 kann durch einen der Umlenkeinrichtung 16 zugeordneten Aktuator 17 verstellbar sein. Die Steuereinrichtung 13 kann konfiguriert sein, den Aktuator 17 abhängig von der durch Auswertung eines Wärmebilds, eines Magnetresonanzbilds, eines Ultraschallbilds oder eines EIT-Bilds ermittelten Lage der Luftkammer 7 anzusteuern.

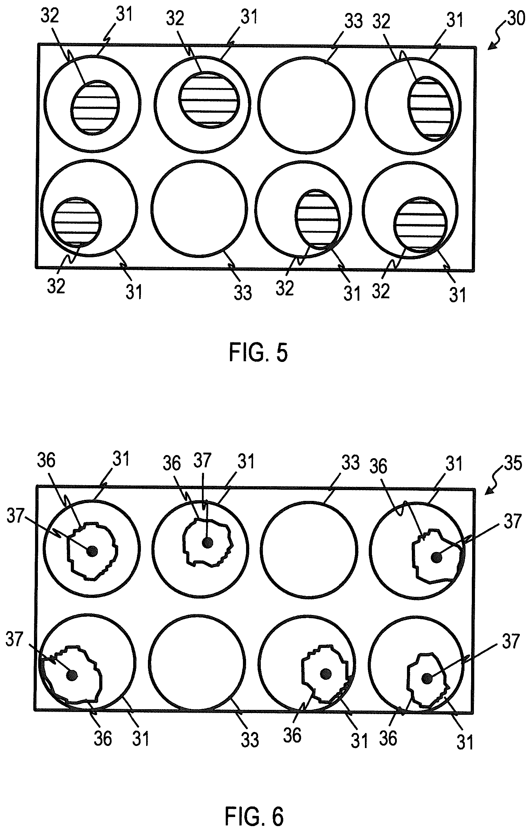

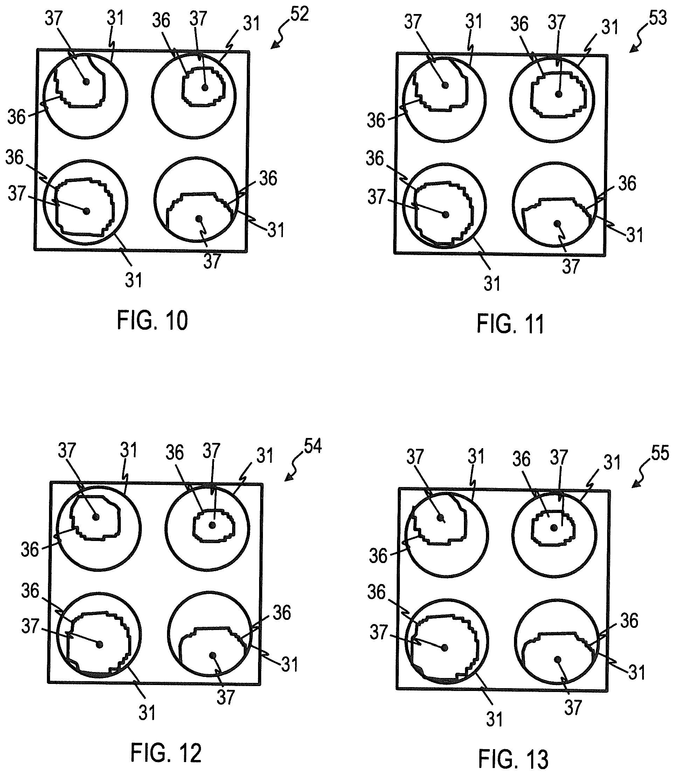

Die Auswertung des Wärmebilds, des Magnetresonanzbilds, des Ultraschallbilds oder des EIT-Bilds zur Ermittlung der Lage der Luftkammer 7 wird unter Bezugnahme auf Figur 5 bis Figur 19 näher beschrieben.

Figur 5 zeigt eine Abbildung 30 mehrerer Vogeleier 31, 33. Die Vogeleier 31 sind mit dem stumpfen Pol nach oben gelagert. Die Vogeleier 33 sind mit dem spitzen Pol nach oben gelagert. Die Vogeleier 31, 33 können in einer Bruthorden oder einer anderen Gruppierung von Vogeleiern angeordnet sein. Die Abbildung 30 kann ein Wärmebild, ein Magnetresonanzbild, ein Ultraschallbild oder ein EIT-Bild sein.

Wenigstens bei einem Teil der mit dem stumpfen Pol nach oben gelagerten Vogeleier 31 ist die Luftkammer 32 im Bereich des stumpfen Pol angeordnet. Die Luftkammer 32 kann in Richtung der Sensoreinrichtung 11 zeigen, wenn die Abbildung erfasst wird. Die genaue Lage und Abmessung der Luftkammer 32 in Bezug auf das jeweilige Vogelei 31 ist bei verschiedenen Vogeleiern 31 typischerweise verschieden. Zur gezielten Bearbeitung der Kalkschale der Vogeleier 31 im Bereich des stumpfen Pols wird die Lage der Luftkammer 32 durch Auswertung der Abbildung 30 ermittelt.

Figur 6 zeigt beispielhaft das Ergebnis einer Auswertung der Abbildung 30. Zur Auswertung wird die Abbildung 30 segmentiert. Verschiedene Segmentierungsmethoden, beispielsweise ein Kantenverfolgungsalgorithmus, eine Wasserscheidentransformation, eine parallele oder sequentielle Kantenextraktion oder andere kantenorientierte Segmentierungsmethoden, oder regionenorientierte Segmentierungsmethoden, wie beispielsweise Energiemethoden, Region Growing, Region-Splitting, Pyramid Linking und Split and Merge, können auf die Abbildung 30 angewandt werden. Durch die Segmentierung kann die Projektion 36 der Luftkammer 32 der Vogeleier 31 in der Bildebene der Sensoreinrichtung 11 identifiziert werden. Da ein Wärmebild verarbeitet wird, kann durch Segmentierung die Projektion der Luftkammer 32 in der Bildebene der Sensoreinrichtung 11 dadurch identifiziert werden, dass sie eine geringere mittlere Temperatur aufweist. Bei einem Magnetresonanzbild kann die Projektion 36 der Luftkammer 32 in der Bildebene der Sensoreinrichtung 11 dadurch identifiziert werden, dass sie eine geringere Dichte von in einem externen Magnetfeld präzedierenden Kernspins aufweist. Bei einem Ultraschallbild kann die Projektion 36 der Luftkammer 32 in der Bildebene der Sensoreinrichtung 11 dadurch identifiziert werden, dass sie eine andere Ausbreitungsgeschwindigkeit für Ultraschallwellen als die mit Flüssigkeit gefüllten Bereiche im Inneren des Vogeleis aufweist, was zu Brechung und/oder Reflexion der Ultraschallwellen führt. Bei einem EIT-Bild kann die Projektion 36 der Luftkammer 32 in der Bildebene der Sensoreinrichtung 11 dadurch identifiziert werden, dass sie eine andere elektromagnetische Impedanz als die mit Flüssigkeit gefüllten Bereiche im Inneren des Vogeleis aufweist.

Nach der Segmentierung kann die Steuereinrichtung 13 einen Flächenmittelpunkt 37 des Segments 36 ermitteln, das der Luftkammer zugeordnet ist. Der Flächenmittelpunkt 37 kann dem Flächenmittelpunkt der Projektion der Luftkammer 32 in der Bildebene der Sensoreinrichtung 11 entsprechen. Der Flächenmittelpunkt 37 kann, muss jedoch nicht unmittelbar durch Berechnung des geometrischen Schwerpunkts des Segments 36 ermittelt werden. Der Flächenmittelpunkt 37 kann beispielsweise auch derart ermittelt werden, dass eine Ellipse in das Segment 36 eingeschrieben oder anderweitig an das Segment 36 angenähert wird, und der Flächenmittelpunkt 37 als Schnittpunkt der Halbachsen der Ellipse bestimmt wird.

Zusätzlich zur Bestimmung des Flächenmittelpunkts 37 kann die Ermittlung der Lage der Luftkammer 32 weitere Schritte aufweisen. Beispielsweise kann wenigstens eine Abmessung des durch Segmentierung ermittelten Segments 36, das der Luftkammer entspricht, bestimmt werden. Alternativ oder zusätzlich kann ein Radius eines Kreises um den Flächenmittelpunkt 37 bestimmt werden, der derart gewählt ist, dass der Kreis vollständig in das der Luftkammer zugeordnete Segment 36 der Abbildung eingeschrieben ist. Durch Ansteuerung einer Bearbeitungseinrichtung 14 in Abhängigkeit von der bestimmten Lage und Größe der Luftkammer 32 kann sichergestellt werden, dass die Kalkschale des Vogeleis nur in demjenigen Bereich bearbeitet wird, an dessen Innenseite die Luftkammer angeordnet ist, ohne dass die innere Membran des Vogeleis durch die Bearbeitung beschädigt wird.

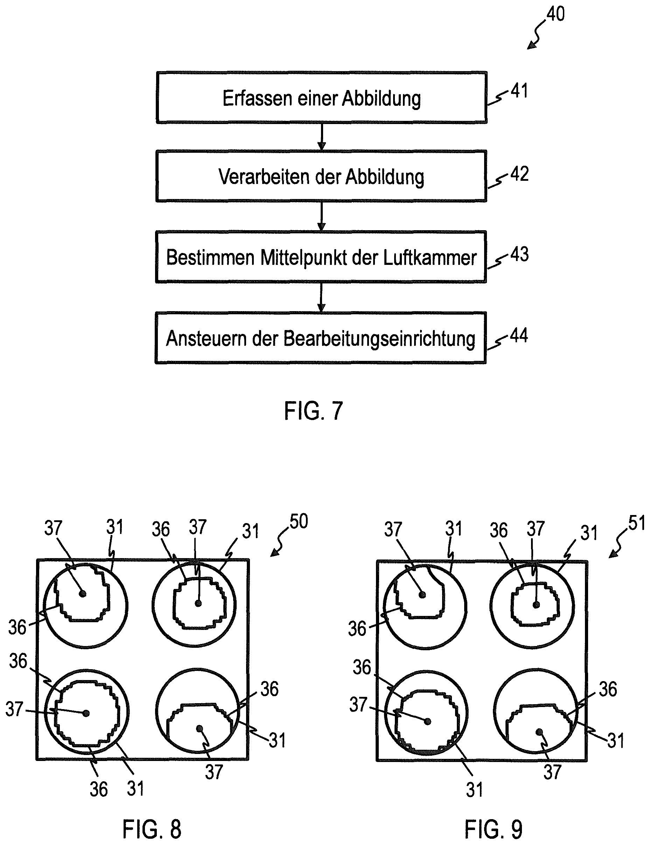

Figur 7 ist ein Flussdiagramm eines Verfahrens 40 nach einem Ausführungsbeispiel. Das Verfahren 40 kann von der Vorrichtung 10 nach einem Ausführungsbeispiel automatisch ausgeführt werden.

Bei Schritt 41 wird eine Abbildung eines Vogeleis erfasst. Zum Erfassen der Abbildung, aus der die Lage der Luftkammer ermittelt wird, wird eine von einer Farbkamera verschiedene Sensoreinrichtung verwendet. Diese Sensoreinrichtung kann insbesondere ein Ausgangssignal oder Ausgangsdaten liefern, die unabhängig von einer Farbe der Kalkschale des abgebildeten Vogeleis sind. Die erfasste Abbildung kann ein Wärmebild, ein Magnetresonanzbild, ein Ultraschallbild oder ein EIT-Bild sein.

Bei Schritt 42 wird die Abbildung ausgewertet. Die Auswertung der Abbildung kann eine Segmentierung aufweisen. Die Auswertung der Abbildung kann eine Filterung und/oder Glättung aufweisen. Die Auswertung kann die Identifizierung einer Projektion der Luftkammer in einer Bildebene der Sensoreinrichtung, die zum Erfassen der Abbildung verwendet wird, aufweisen.

Bei Schritt 43 wird ein Mittelpunkt der Luftkammer ermittelt. Dazu kann ein Flächenmittelpunkt der Projektion der Luftkammer in die Bildebene der Sensoreinrichtung bestimmt werden. Falls die Sensoreinrichtung zur Erfassung einer dreidimensionalen Abbildung eingerichtet ist, wie dies beispielsweise bei dreidimensional ortsaufgelösten Magnetresonanztechniken oder zahlreichen Ultraschall-Bildgebungsmethoden der Fall ist, kann auch ein Volumenmittelpunkt der Luftkammer ermittelt werden.

Bei Schritt 43 kann optional wenigstens eine Abmessung der Luftkammer ermittelt werden. Beispielsweise kann ein Radius eines Kreises ermittelt werden, der in die Projektion der Luftkammer in die Bildebene der Sensoreinrichtung ein beschrieben werden kann.

Bei Schritt 44 wird die Bearbeitungseinrichtung zum Bearbeiten der Kalkschale des Vogeleis abhängig von der ermittelten Lage der Luftkammer angesteuert. Beispielsweise kann eine Schwächungslinie derart in die Kalkschale eingebracht werden, dass die Kalkschale nur in demjenigen Bereich geschwächt wird, der die Luftkammer überdeckt, ohne die innere Membran zu beschädigen.

Die Vorrichtungen und Verfahren nach Ausführungsbeispielen erlauben eine robuste und zuverlässige Bestimmung der Lage der Luftkammer zur Ansteuerung einer Bearbeitungseinrichtung. Dies wird für die Verwendung einer Wärmebildkamera beispielhaft unter Bezugnahme auf Figur 8 bis Figur 19 näher beschrieben.

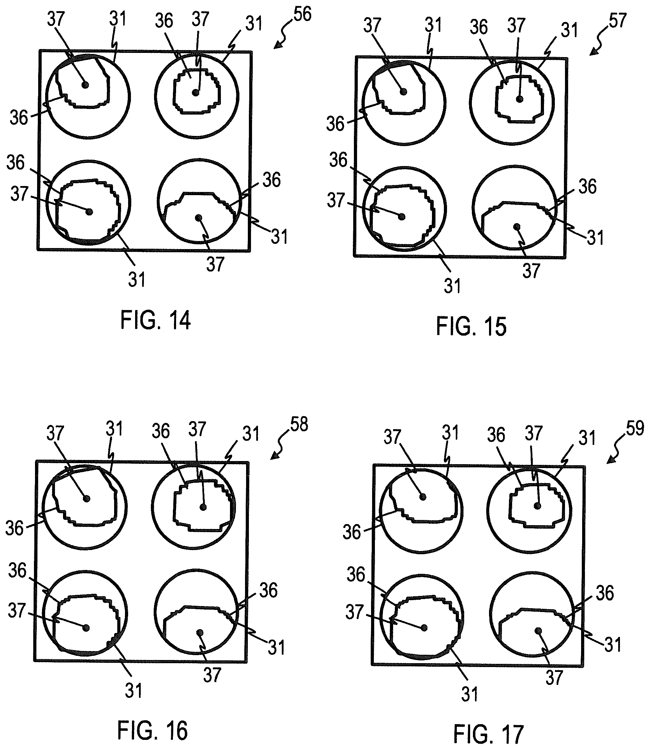

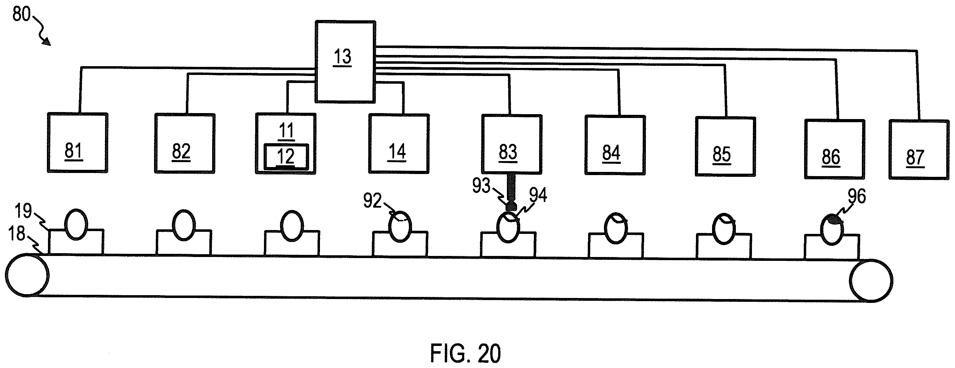

Figur 8 bis Figur 17 zeigen jeweils Wärmebilder 50-61, die für dieselben Vogeleier 31 zu unterschiedlichen Zeiten nach Entnahme aus einer Bruteinrichtung mit einer Wärmebildkamera erfasst wurden. Dabei sind jeweils die durch Segmentierung ermittelte Projektion 36 der Luftkammer in der Bildebene der Wärmebildkamera sowie der Flächenmittelpunkt 37 der Projektion 36 dargestellt. Das Wärmebild 50 zeigt die segmentierte Abbildung unmittelbar nach Entnahme aus der Bruteinrichtung. Das Wärmebild 51 zeigt die segmentierte Abbildung fünf Minuten nach Entnahme aus der Bruteinrichtung. Das Wärmebild 52 zeigt die segmentierte Abbildung zehn Minuten nach Entnahme aus der Bruteinrichtung. Das Wärmebild 53 zeigt die segmentierte Abbildung 15 Minuten nach Entnahme aus der Bruteinrichtung. Das Wärmebild 54 zeigt die segmentierte Abbildung 20 Minuten nach Entnahme aus der Bruteinrichtung. Das Wärmebild 55 zeigt die segmentierte Abbildung 25 Minuten nach Entnahme aus der Bruteinrichtung das Wärmebild 56 zeigt die segmentierte Abbildung 30 Minuten nach Entnahme aus der Bruteinrichtung. Das Wärmebild 57 zeigt die segmentierte Abbildung 35 Minuten nach Entnahme aus der Bruteinrichtung. Das Wärmebild 58 zeigt die segmentierte Abbildung 40 Minuten nach Entnahme aus der Bruteinrichtung. Das Wärmebild 59 zeigt die segmentierte Abbildung 45 Minuten nach Entnahme aus der Bruteinrichtung.

Wie aus Figur 8 bis Figur 17 deutlich wird, verändert sich die durch Segmentierung des Wärmebilds jeweils bestimmte Projektion 36 der Luftkammer in der Bildebene der Wärmebildkamera nur geringfügig als Funktion der Zeit seit Entnahme aus der Bruteinrichtung. Selbst für eine längere Zeitdauer zwischen der Entnahme aus der Bruteinrichtung und der Erfassung des Wärmebilds kann aus dem Wärmebild die Lage der Luftkammer somit zuverlässig ermittelt werden. Auch wenn sich die Ränder der Segmente, die der Luftkammer zugeordnet sind, geringfügig verschieben, bleiben die jeweils bestimmten Flächenmittelpunkt des 37 im Wesentlichen an derselben Stelle.

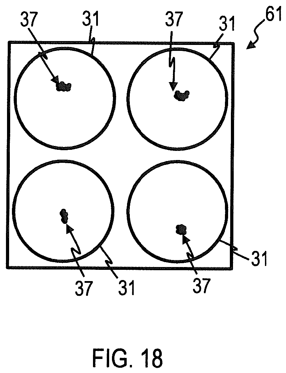

Figur 18 ist eine Darstellung 61, die alle aus den Wärmebildern 51-59 gewonnenen Flächenmittelpunkte 37 de darstellt. Wie aus Figur 18 deutlich wird, ist die Lage der ermittelten Flächenmittelpunkte 37 für Zeiten unmittelbar nach Entnahme aus der Bruteinrichtung bis 45 Minuten nach Entnahme aus zu Bruteinrichtung konsistent und unterliegt nur einer geringfügigen örtlichen Abweichung, die klein im Vergleich zum Radius des Vogeleis ist.

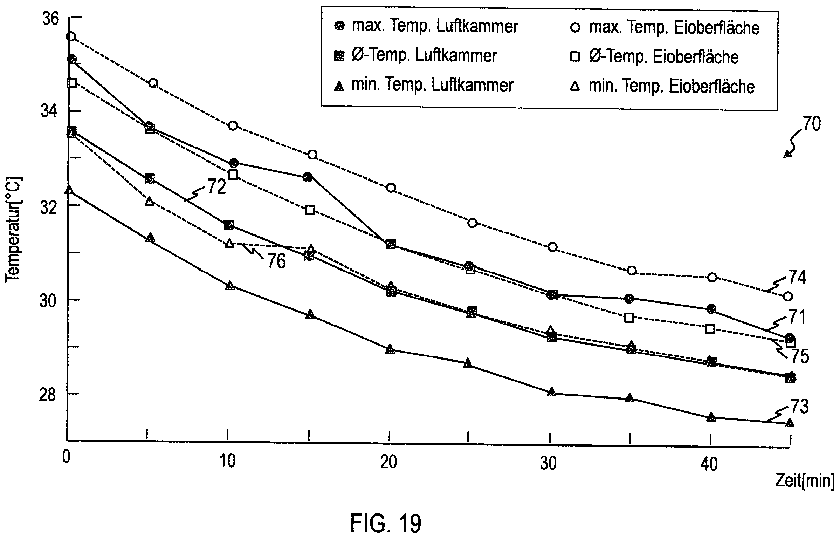

Figur 19 ist eine grafische Darstellung der Temperatur der Kalkschale im Bereich der Luftkammer sowie im die Luftkammer umgebenden Bereich der Kalkschale für verschiedene Zeiten ab Entnahme der Vogeleier aus einer Bruteinrichtung. Eine maximale Temperatur der Kalkschale im Bereich der Luftkammer ist durch gefüllte Kreise und eine durchgezogene Linie 71 dargestellt. Eine minimale Temperatur der Kalkschale im Bereich der Luftkammer ist durch gefüllte Dreiecke und eine durchgezogene Linie 73 dargestellt. Eine gemittelte Temperatur der Kalkschale im Bereich der Luftkammer ist durch gefüllte Quadrate und eine durchgezogene Linie 72 dargestellt. Eine maximale Temperatur der gesamten Kalkschale ist durch nicht gefüllte Kreise und eine unterbrochene Linie 74 dargestellt. Eine minimale Temperatur der gesamten Kalkschale ist durch nicht gefüllte Dreiecke und eine unterbrochene Linie 76 dargestellt. Eine gemittelte Temperatur der gesamten Kalkschale ist durch nicht gefüllte Quadrate und eine unterbrochene Linie 75 dargestellt.

Wie aus Figur 19 ersichtlich ist, liegt die mittlere Temperatur 72 der Kalkschale im Bereich der Luftkammer deutlich unterhalb der mittleren Temperatur 75 der gesamten Kalkschale. Dies erlaubt eine zuverlässige Bestimmung der Lage der Luftkammer, die auch robust gegenüber der seit Entnahme aus der Bruteinrichtung verstrichenen Zeit ist, sofern diese nicht zu lang ist. Für die in Figur 19 dargestellten Zeiten von 0 bis 45 Minuten seit Entnahme aus der Bruteinrichtung ist eine zuverlässige und robuste Ermittlung der Lage der Luftkammer durch Segmentierung eines Wärmebilds möglich.

Die Vorrichtung und das Verfahren nach Ausführungsbeispielen können zusätzliche Einheiten zur Beeinflussung des Vogeleis einsetzen. Insbesondere können die Vorrichtung und das Verfahren nach Ausführungsbeispielen derart konfiguriert sein, dass die Kalkschale nach der Bearbeitung durch die Bearbeitungseinrichtung durch eine Öffnungseinrichtung lokal im Bereich der Luftkammer aufgebrochen wird. Durch eine Messeinrichtung kann wenigstens eine Messung an dem geöffneten Vogelei ausgeführt werden, um beispielsweise ein Geschlecht des Embryos in dem Vogelei zu bestimmen, wie nachfolgend ausführlicher beschrieben wird.

Figur 20 ist eine schematische Darstellung einer Vorrichtung 80 nach einem Ausführungsbeispiel, die eine Sensoreinrichtung 11, eine Steuereinrichtung 13 und eine Bearbeitungseinrichtung 14 aufweist. Die Sensoreinrichtung 11, Steuereinrichtung 13 und Bearbeitungseinrichtung 14 können wie unter Bezugnahme auf Figur 1 bis Figur 19 beschrieben ausgestaltet sein.

Die Vorrichtung 80 kann eine Desinfektionseinrichtung 81 zum Desinfizieren zumindest des Bereiches des stumpfen Pols der Vogeleier aufweisen.

Die Vorrichtung 80 kann eine Detektionseinrichtung 82 zum Detektieren der Abmessungen des Vogeleis aufweisen. Die Detektionseinrichtung 82 kann einen Bildsensor aufweisen, dem optional ein Abstandssensor oder ein Triangulationssensor zugeordnet sein kann. Die Steuereinrichtung 13 kann konfiguriert sein, die durch die Detektionseinrichtung 82 detektierte Abmessung des Vogeleis mit der durch Auswertung des Wärmebilds, des Magnetresonanzbilds, des Ultraschallbilds oder des EIT-Bilds ermittelten Lage der Luftkammer zu kombinieren, um zu bestimmen, an welchen Punkten oder in welchen Bereichen auf der Kalkschale des Vogeleis eine Bearbeitung erfolgen soll.

Die Vorrichtung 80 kann eine Öffnungseinrichtung 83 aufweisen. Die Öffnungseinrichtung 83 kann konfiguriert sein, die Kalkschale des Vogeleis entlang einer durch die Bearbeitungseinrichtung 14 eingebrachten Schwächungslinie 92 aufzubrechen. Die Öffnungseinrichtung 83 kann konfiguriert sein, einen Deckel 94 der aufgebrochenen Kalkschale, der innerhalb der Schwächungslinie 92 liegt, von dem Vogelei abzuheben. Die Öffnungseinrichtung 83 kann ein Element 93 zum mechanischen Einwirken auf die Kalkschale 3 aufweisen.

Die Vorrichtung 80 kann eine weitere Sensoreinrichtung 84 aufweisen, die der Bearbeitungseinrichtung 14 und optional der Öffnungseinrichtung 83 nachgeordnet ist. Die weitere Sensoreinrichtung 84 kann konfiguriert sein, eine Lage des Embryos in dem geöffneten Vogelei zu bestimmen. Die weitere Sensoreinrichtung 84 kann insbesondere konfiguriert sein, ein Bild zu erfassen, aus dem die Steuereinrichtung 13 bestimmen kann, an welcher Position unterhalb der inneren Membran der Embryo innerhalb des geöffneten Vogeleis positioniert ist.

Die Vorrichtung 80 kann eine Messeinrichtung 85 aufweisen. Die Messeinrichtung 85 kann für wenigstens eine Messung an dem geöffneten Vogelei zum Bestimmen eines Geschlechts des Embryos konfiguriert sein. Die Messeinrichtung 85 kann für wenigstens eine spektroskopische Untersuchung an dem Embryo innerhalb des Vogeleis konfiguriert sein. Die Messeinrichtung 85 kann eine absorptionsspektroskopische Messung oder eine andere spektroskopische Messung, beispielsweise eine Raman-Spektroskopie, an dem Embryo vornehmen. Die Messeinrichtung 85 kann eine Infrarot (IR)-Raman-Spektroskopie an dem Embryo vornehmen. Die Messeinrichtung 85 kann alternativ oder zusätzlich konfiguriert sein, eine Fluoreszenspektroskopie und/oder Absorptionsspektroskopie auszuführen. Das erfasste Spektrum oder die erfassten Spektren können ausgewertet werden, um das Geschlecht des Embryos zu bestimmen. Dazu kann beispielsweise die Steuereinrichtung 13 oder eine davon separate Auswerteschaltung das erfasste Spektrum verarbeiten. Die Verarbeitung des erfassten Spektrums kann eine Hauptkomponentenanalyse oder eine Clusteranalyse aufweisen. Beispielsweise kann das Spektrum wie in der WO 2017/017277 A1 beschrieben ausgewertet werden.

Die Steuereinrichtung 13 kann konfiguriert sein, die Messeinrichtung 85 abhängig von der Lage des Embryos im Vogelei anzusteuern, die abhängig von einem Ausgangssignal oder Ausgangsdaten der Sensoreinrichtung 84 bestimmt wird.

Die Vorrichtung 80 kann eine Verschließeinrichtung 86 zum Verschließen der Öffnung in der Kalkschale des Vogeleis aufweisen. Die Verschließeinrichtung 86 kann konfiguriert sein, ein Verschlusselement 96 aus biokompatiblem Material über der Öffnung zu positionieren, um die Kalkschale des Vogeleis zu verschließen.

Die Vorrichtung 80 kann weitere Einrichtungen aufweisen, die abhängig von der mit der Sensoreinrichtung 11 erfassten Abbildung angesteuert werden. Beispielsweise kann die Vorrichtung 80 eine Sortiereinrichtung 87 zum Aussortieren von nicht verwendbaren Vogeleiern oder nicht verwendbaren Gruppen von Vogeleiern oder zum anderweitigen Sortieren von Vogeleiern aufweisen. Die Steuereinrichtung 13 kann konfiguriert sein, die Sortiereinrichtung 87 abhängig von einem Wärmebild, einem Magnetresonanzbild, einem Ultraschallbild oder einem EIT-Bild anzusteuern, das mit der Sensoreinrichtung 11 erfasst wird. Beispielsweise kann die Steuereinrichtung 13 abhängig von der Temperaturinformation eines Wärmebilds ermitteln, ob ein Vogelei für einen nicht akzeptablen Zeitraum außerhalb einer Bruteinrichtung positioniert war. Alternativ oder zusätzlich kann die Steuereinrichtung 13 konfiguriert sein, abhängig von Temperaturinformation des Wärmebilds wenigstens einen Teil der weiteren Prozessführung des Brutprozesses zu steuern.

Bei der Vorrichtung nach einem Ausführungsbeispiel kann nicht nur die Bearbeitungseinrichtung 14 zum Einbringen einer Schwächungslinie in die Kalkschale, sondern auch die Öffnungseinrichtung 83 in Abhängigkeit von der durch Auswertung eines Wärmebilds, eines Magnetresonanzbilds, eines Ultraschallbilds oder eines EIT-Bilds ermittelten Lage der Luftkammer angesteuert werden.

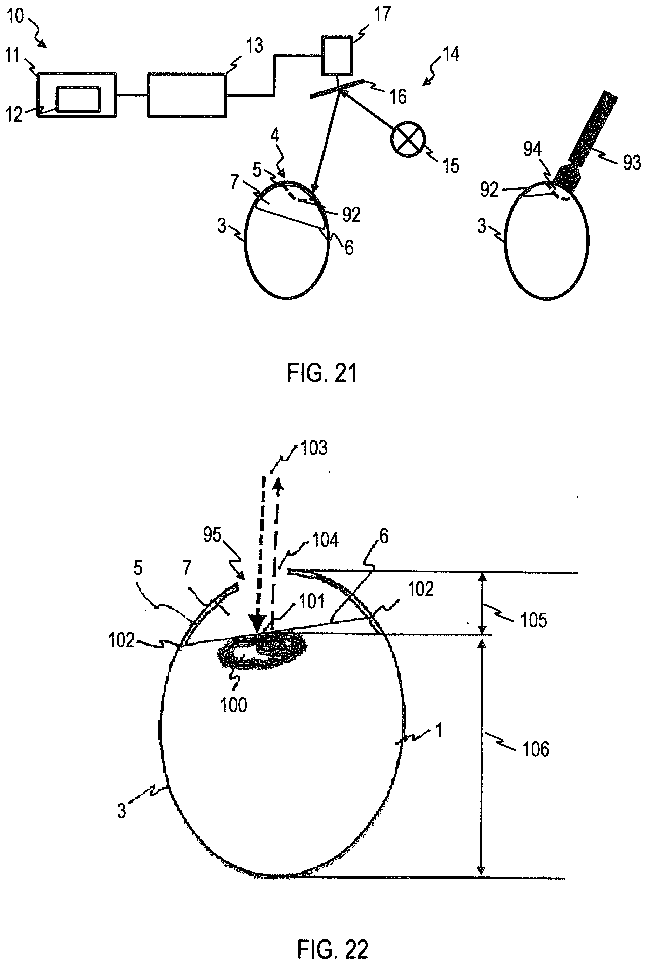

Figur 21 zeigt eine schematische Darstellung einer Vorrichtung 10 gemäß einem Ausführungsbeispiel. Die Vorrichtung 10 weist eine Sensoreinrichtung 11, eine Steuereinrichtung 13, eine Bearbeitungseinrichtung 14 zum Bearbeiten der Kalkschale 3 des Vogeleis und eine Öffnungseinrichtung mit einem Element 93 zum Aufbrechen der Kalkschale 3 auf. Wie unter Bezugnahme auf Figur 1 bis Figur 20 beschrieben wurde, kann die Lage der Luftkammer 7 in dem Vogelei durch Auswertung eines Wärmebilds, eines Magnetresonanzbilds, eines Ultraschallbilds, eines EIT-Bilds oder einer anderen Abbildung des Vogeleis, die nicht mit einer im sichtbaren Spektralbereich farbensensitiven Farbkamera erfasst wurde, bestimmt werden. Abhängig von der Lage und der Abmessung der Luftkammer 7 kann die Bearbeitungseinrichtung 14 so angesteuert werden, dass eine Schwächungslinie 92 im Bereich der Luftkammer 7 in die Kalkschale 3 eingebracht wird. Die Öffnungseinrichtung kann ebenfalls abhängig von der durch Auswertung des Wärmebilds, des Magnetresonanzbilds, des Ultraschallbilds oder des EIT-Bilds ermittelten Lage der Luftkammer 7 so angesteuert werden, dass das Element 93 die Kalkschale 3 an dem innerhalb der Schwächungslinie 92 definierten Deckel 94 der Kalkschale berührt.

Figur 22 zeigt beispielhaft ein Vogelei 1 mit darin enthaltenem Embryo 100. Eine Öffnung 95 kann durch die Vorrichtung nach einem Ausführungsbeispiel im Bereich der Luftkammer 7 eingebracht werden. Der Embryo 100 ist unterhalb der inneren Membran 6 in dem Vogelei 1 positioniert. Eine Bindungsgrenze zwischen der Luftkammer 7 und der inneren Membran 6 ist durch die Bezugszeichen 102 dargestellt. Die Vorrichtung nach einem Ausführungsbeispiel kann konfiguriert sein, die Lage des Embryos 100 in dem Vogelei 1 nach dem Öffnen der Kalkschale 3 zu bestimmen. Dazu kann ein Abstand 105 des Embryos 100 von einem oberen Zenit des Vogeleis 1 am stumpfen Pol bestimmt werden. Alternativ oder zusätzlich kann ein Abstand 106 des Embryos 100 von einem unteren Zenit des Vogeleis 1 am spitzen Pol bestimmt werden.

Für eine Untersuchung des Embryos 100, beispielsweise zur Geschlechtsbestimmung, kann ein Anregungsstrahl 103 für eine spektroskopische Untersuchung so durch die Öffnung 95 in das Innere des Vogeleis 1 gelenkt werden, dass er an der inneren Membran 6 an einer Position 101 benachbart dem Embryo 100 auftrifft. Der Anregungsstrahl 103 kann gezielt auf ein Blutgefäß des Embryos 100 gelenkt werden. Raman-Streulicht 104 oder Fluoreszenzlicht 104 kann nach dem Verlassen des Vogeleis durch die Öffnung 95 wellenlängenaufgelöst erfasst werden. Anstelle einer Raman-Spektroskopie können auch andere spektroskopische Methoden, beispielsweise eine Absorptionsspektroskopie oder eine Fluoreszenzspektroskopie, eingesetzt werden.

Mit der Vorrichtung und dem Verfahren nach Ausführungsbeispielen können charakteristische Spektren embryonaler Blutgefäße zur Bestimmung des Geschlechts des sich entwickelnden Vogel embryos 100 aufgenommen werden. Die Spektren können mithilfe bekannter Verfahren der Datenanalyse ausgewertet werden, um das Geschlecht des sich entwickelnden Vogelembryos zu bestimmen.

Beispielsweise kann eine Hauptkomponentenanalyse oder eine Clusteranalyse zur Auswertung der Spektren verwendet werden, um das Geschlecht des sich entwickelnden Vogelembryos zu bestimmen. Bei der Clusteranalyse werden im Wesentlichen zwei Verfahren miteinander kombiniert, nämlich die Hauptkomponentenanalyse und eine K-Nächste-Nachbarn-Klassifikation.

Die Hauptkomponentenanalyse dient der Strukturierung und Vereinfachung der aufgenommenen Daten. Dabei wird das erfasste Spektrum durch eine geringere Anzahl von Linearkombinationen dargestellt, als das Signal Messwerte hat. Dieser Schritt unterdrückt das Rauschen bei der Messung und macht die gemessenen Signale vergleichbarer. Nachdem das Signal in seine Hauptkomponenten zerlegt ist, ist es möglich, mit diesen Komponenten einen mathematischen Raum aufzustellen, d.h. die Parameter jeder Linearkombination einer Hauptkomponente entsprechen einem Punkt in einem zweidimensionalen Raum, bei dem die Achsen alle möglichen Kombinationen der Parameter einer möglichen Linearkombination sind. Es bildet sich somit eine Punktwolke. Diese Punktwolke wird im nächsten Schritt für die K-Nächste-Nachbarn-Klassifikation verwendet.

Bei ausgewerteten Spektren entspricht die K-Nächste-Nachbarn-Klassifikation k=1. Das stellt einen Spezialfall dar, und wird als Voronoi-Diagramm bezeichnet. Die gewonnene Punktwolke wird in Regionen unterteil. Jeder Punkt bildet dabei das Zentrum einer Region. Auf dieses Konstrukt lassen sich verschiedene Metriken anwenden, z.B. die euklidische Distanz der Zentren zueinander oder die Flächengröße von bestimmten Regionen. Bestimmte Kombinationen von Zentren und Regionen sind spezifisch für ein Geschlecht des sich entwickelnden Vogelembryos und unterscheiden sich signifikant für männliche und weibliche Embryos.

Auch wenn an dieser Stelle beispielhaft die Clusteranalyse beschrieben wurde, können andere Datenanalyseverfahren zur Bestimmung des Geschlechts des sich entwickelnden Embryos 100 auf Basis der gewonnenen Signale verwendet werden. Die genannten bekannten Datenanalyseverfahren sind auch auf die mit anderen Analyseverfahren, wie Fluoreszenzspektroskopie oder weitere spektroskopische Verfahren anwendbar, um die erfassten Spektren auszuwerten.

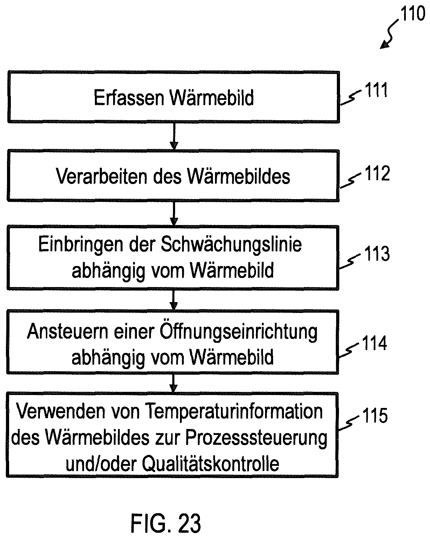

Bei den Vorrichtungen und Verfahren nach Ausführungsbeispielen können das zur Ermittlung der Lage der Luftkammer ausgewertete Wärmebild, Magnetresonanzbild, Ultraschallbild oder EIT-Bild auch für weitere Steuer- oder Kontrollfunktionen eingesetzt werden. Dies wird unter Bezugnahme auf Figur 23 beispielhaft für ein Wärmebild beschrieben.

Figur 23 ist ein Flussdiagramm 110 eines Verfahrens nach einem Ausführungsbeispiel. Das Verfahren kann von der Vorrichtung nach einem Ausführungsbeispiel ausgeführt werden.

Bei Schritt 111 wird ein Wärmebild des Vogeleis erfasst. Das Wärmebild kann mit einer Wärmebildkamera erfasst werden, die im sichtbaren Bereich des elektromagnetischen Spektrums nicht sensitiv ist oder jedenfalls keine Farbauflösung im sichtbaren Bereich des elektromagnetischen Spektrums aufweist.

Bei Schritt 112 wird das Wärmebild verarbeitet. Die Verarbeitung des Wärmebilds kann eine Segmentierung aufweisen. Mit der Segmentierung kann eine Projektion der Luftkammer in der Bildebene der Wärmebildkamera identifiziert werden.

Bei Schritt 113 wird eine Bearbeitungseinrichtung zum Bearbeiten der Kalkschale des Vogeleis abhängig von der ermittelten Lage der Luftkammer angesteuert. Die Bearbeitungseinrichtung kann eine Schwächungslinie in die Kalkschale derart einbringen, dass die Schwächungslinie vollständig an dem die Luftkammer überdeckenden Bereich der Kalkschale positioniert ist und einen Abstand zur inneren Membran des Vogeleis aufweist.

Bei Schritt 114 kann eine Öffnungseinrichtung zum Aufbrechen der Kalkschale ebenfalls in Abhängigkeit von der durch Auswertung des Wärmebilds ermittelten Lage der Luftkammer angesteuert werden. Bei weiteren Ausgestaltungen kann die Öffnungseinrichtung unabhängig von dem Wärmebild angesteuert werden, beispielsweise indem eine separate Farbaufnahme des Vogeleis nach dem Einbringen der Schwächungslinie aufgenommen und ausgewertet wird, um die Position der Schwächungslinie zu verifizieren.

Bei Schritt 115 kann in dem Wärmebild enthaltene Temperaturinformation für Kontroll- und/oder Steuerfunktionen verwendet werden. Beispielsweise kann anhand der Temperatur des Vogeleis in dem Wärmebild bestimmt werden, ob die Prozessführung für das Vogelei bisher optimal oder wenigstens in einem akzeptablen Temperaturbereich vorgenommen wurde. Abhängig von der Temperaturinformation können Vogeleier automatisch aussortiert oder eine Warnung an einen Benutzer ausgegeben werden. Alternativ oder zusätzlich kann wenigstens ein Teil der weiteren Prozessführung des Brutprozesses in Abhängigkeit von in dem Wärmebild enthaltener Temperaturinformation gesteuert werden.

Während unter Bezugnahme auf Figur 23 ein Verfahren beschrieben wurde, bei dem ein Wärmebild verwendet wird, um sowohl die Lage der Luftkammer zu ermitteln als auch eine Qualitätskontrolle auszuführen und/oder die Prozessführung des Brutprozesses zu steuern, können auch Magnetresonanzbilder, Ultraschallbilder oder EIT-Bilder verwendet werden, um eine Qualitätskontrolle auszuführen und/oder die Prozessführung des Brutprozesses zu steuern.

Während Ausführungsbeispiele unter Bezugnahme auf die Figuren beschrieben wurden, können bei weiteren Ausführungsbeispielen Abwandlungen oder Modifizierungen verwendet werden. Beispielsweise muss die Bearbeitungseinrichtung nicht notwendig konfiguriert sein, eine Schwächungslinie in die Kalkschale des Vogeleis einzubringen. Auch andere auf die Kalkschale angewandte Bearbeitungstechniken, beispielsweise zur Durchführung einer Bestimmung des Geschlechts des Embryos in dem Vogelei, können in Abhängigkeit von einer Auswertung eines Wärmebilds, Magnetresonanzbilds, Ultraschallbilds oder EIT-Bilds ausgeführt werden.

## Patentansprüche

1. Verfahren zum Bearbeiten einer Kalkschale (3) eines Vogeleis (1) mit darin enthaltenem Embryo (100) im Bereich des stumpfen Pols (4) des Vogeleis (1), insbesondere zum Einbringen einer Schwächungslinie in die Kalkschale (3) im Bereich des stumpfen Pols (4), aufweisend die Schritte:
Ermitteln einer Lage einer Luftkammer (7), die zwischen einer inneren Membran (6) und einer äußeren Membran (5) des Vogeleis (1) im Bereich des stumpfen Pols (4) angeordnet ist, wobei das Ermitteln der Lage der Luftkammer (7) aufweist:
Erfassen einer Abbildung (30; 50-59) des Vogeleis (1) unter Verwendung einer Sensoreinrichtung (11), deren Ausgangssignal oder Ausgangsdaten im Wesentlichen unabhängig von einer Farbe der Kalkschale (3) sind, wobei die Abbildung (30; 50-59) ein Wärmebild (50-59) ist, und Auswerten der Abbildung (30; 50-59) zum Ermitteln der Lage der Luftkammer (7), wobei das Auswerten der Abbildung (30; 50-59) eine Segmentierung der Abbildung (30; 50-59) aufweist, wobei die Segmentierung eine kantenorientierte Segmentierungsmethode oder eine regionenorientierte Segmentierungsmethode aufweist, wobei eine Projektion (32) der Luftkammer (7) in einer Bildebene der Sensoreinrichtung (11) dadurch identifiziert wird, dass sie eine geringere mittlere Temperatur aufweist; und
Ansteuern einer Bearbeitungseinrichtung (14) zum Bearbeiten der Kalkschale (3) im Bereich des stumpfen Pols (4) abhängig von der durch das Auswerten der Abbildung (30; 50-59) ermittelten Lage der Luftkammer (7), so dass die Kalkschale (3) in einem Abstand von der inneren Membran (6) des Vogeleis (1) bearbeitet wird.

2. Verfahren nach Anspruch 1, wobei die Segmentierung der Abbildung (30; 50-59) einen Kantenverfolgungsalgorithmus, eine Wasserscheidentransformation, eine parallele Kantenextraktion oder eine sequentielle Kantenextraktion aufweist.

3. Verfahren nach Anspruch 1, wobei die Segmentierung der Abbildung (30; 50-59) eine Energiemethode, Region Growing, Region-Splitting, Pyramid Linking oder Split and Merge aufweist.

4. Verfahren nach einem der vorhergehenden Ansprüche, wobei durch die Segmentierung eine Projektion der Luftkammer (7) in eine Bildebene der Sensoreinrichtung (11) identifiziert wird.

5. Verfahren nach einem der vorhergehenden Ansprüche, wobei das Auswerten der Abbildung (30; 50-59) aufweist:
Bestimmen eines Flächenmittelpunkts (37) oder Volumenmittelpunkts eines durch die Segmentierung ermittelten Segments (36) der Abbildung (30; 50-59).

6. Verfahren nach einem der vorhergehenden Ansprüche, wobei durch das Ansteuern der Bearbeitungseinrichtung (14) eine Richtung, aus der die Bearbeitungseinrichtung (14) auf die Kalkschale (3) einwirkt, abhängig von der durch das Auswerten der Abbildung (30; 50-59) ermittelten Lage der Luftkammer (7) beeinflusst wird.

7. Verfahren nach Anspruch 6, wobei die Bearbeitungseinrichtung (14) eine Quelle (15) elektromagnetischer Strahlung und eine steuerbare Umlenkeinrichtung (16, 17) für von der Quelle (15) erzeugte elektromagnetische Strahlung aufweist, wobei die Umlenkeinrichtung (16, 17) abhängig von der durch das Auswerten der Abbildung (30; 50-59) ermittelten Lage der Luftkammer (7) angesteuert wird, um eine Schwächungslinie (92) in die Kalkschale (3) einzubringen.

8. Verfahren nach einem der vorhergehenden Ansprüche,
wobei die Sensoreinrichtung (11) eine Wärmebildkamera (12) zum Erfassen eines Wärmebilds (50-59) aufweist, und wobei das Verfahren den Schritt aufweist:
Verwenden von aus dem Wärmebild (50-59) gewonnener Temperaturinformation zur Qualitätssicherung und/oder zur Prozessführung wenigstens eines Abschnitts eines Brutprozesses des Vogeleis (1).

9. Verfahren zur Geschlechtsbestimmung eines in einem bebrüteten Vogelei (1) enthaltenen Embryos (100) anhand einer Messung am stumpfen Pol (4) des Vogeleis (1), aufweisend die Schritte:
Einbringen einer Schwächungslinie (92) in die Kalkschale (3) des Vogeleis (1) mit dem Verfahren nach einem der vorhergehenden Ansprüche,
Abheben eines durch die Schwächungslinie definierten Bereichs (94) der Kalkschale (3) zum Öffnen des Vogeleis (1),
Ausführen wenigstens einer Messung an dem geöffneten Vogelei zum Bestimmen des Geschlechts des Embryos (100) und
Verschließen der geöffneten Kalkschale (3) des Vogeleis (1) durch Aufsetzen eines Verschlussmaterials (96).

10. Vorrichtung (10; 80) zum Bearbeiten einer Kalkschale (3) eines Vogeleis (1) mit darin enthaltenem Embryo (100) im Bereich des stumpfen Pols (4) des Vogeleis (1), insbesondere zum Einbringen einer Schwächungslinie in die Kalkschale (3) im Bereich des stumpfen Pols (4), aufweisend:
eine Sensoreinrichtung (11), deren Ausgangssignal oder Ausgangsdaten von einer Farbe der Kalkschale (3) im Wesentlichen unabhängig sind, zum Erfassen einer Abbildung (30; 50-59) des Vogeleis (1), wobei die Sensoreinrichtung (11) eine Wärmebildkamera (12) aufweist,
eine Steuereinrichtung (13) zum Auswerten der Abbildung (30; 50-59), um eine Lage einer Luftkammer (7), die zwischen einer inneren Membran (6) und einer äußeren Membran (5) des Vogeleis (1) im Bereich des stumpfen Pols (4) angeordnet ist, zu bestimmen, und
eine Bearbeitungseinrichtung (14), die von der Steuereinrichtung (13) steuerbar ist, zum Bearbeiten der Kalkschale (3) im Bereich des stumpfen Pols (4) abhängig von der durch die Steuereinrichtung ermittelten Lage der Luftkammer (7), um die Kalkschale (3) in einem Abstand von der inneren Membran (6) des Vogeleis (1) zu bearbeiten,
wobei die Steuereinrichtung (13) konfiguriert ist, die Abbildung (30; 50-59) unter Verwendung einer kantenorientierten Segmentierungsmethode oder einer regionenorientierten Segmentierungsmethode zu segmentieren, wobei die Steuereinrichtung (13) konfiguriert ist, eine Projektion (32) der Luftkammer (7) in einer Bildebene der Sensoreinrichtung (11) dadurch zu identifizieren, dass sie eine geringere mittlere Temperatur aufweist.

11. Vorrichtung nach Anspruch 10, wobei die Steuereinrichtung (13) konfiguriert ist, die Abbildung (30; 50-59) unter Verwendung eines Kantenverfolgungsalgorithmus, einer Wasserscheidentransformation, einer parallelen Kantenextraktion oder einer sequentiellen Kantenextraktion zu segmentieren.

12. Verfahren nach Anspruch 10, wobei die Steuereinrichtung (13) konfiguriert ist, die Abbildung (30; 50-59) unter Verwendung einer Energiemethode, Region Growing, Region-Splitting, Pyramid Linking oder Split and Merge zu segmentieren.

13. Vorrichtung nach einem der Ansprüche 10 bis 12, wobei die Steuereinrichtung (13) konfiguriert ist, einen Flächenmittelpunkt (37) oder Volumenmittelpunkt eines durch die Segmentierung der Abbildung (30; 50-59) ermittelten Segments (36) der Abbildung (30; 50-59) zu ermitteln.

14. Vorrichtung nach einem der Ansprüche 10 bis 13,
wobei die Bearbeitungseinrichtung (14) konfiguriert ist, eine Schwächungslinie (92) in die Kalkschale (3) einzubringen, und wobei die Vorrichtung (10; 80) ferner aufweist:
eine Öffnungseinrichtung (83) zum Abheben eines durch die Schwächungslinie (92) definierten Bereichs (94) der Kalkschale (3) zum Öffnen des Vogeleis (1),
eine Messeinrichtung (85) zum Ausführen wenigstens einer Messung an dem geöffneten Vogelei zum Bestimmen eines Geschlechts des Embryos (100) und
eine Verschließeinrichtung (86) zum Verschließen der geöffneten Kalkschale (3) des Vogeleis (1).

15. Vorrichtung nach einem der Ansprüche 10 bis 14, wobei die Vorrichtung (10; 80) zur Durchführung des Verfahrens nach einem der Ansprüche 1 bis 9 konfiguriert ist.

## Claims

1. A method for processing a calcified shell (3) of an avian egg (1) containing an embryo (100) therein in the region of the blunt end (4) of the avian egg (1), particularly for generating a weakening line in the calcified shell (3) in the region of the blunt end (4), comprising the steps of:
determining a position of an air cell (7) which is disposed between an inner membrane (6) and an outer membrane (5) of the avian egg (1) in the region of the blunt end (4), wherein the determination of the position of the air cell (7) comprises:
capturing an image (30; 50-59) of the avian egg (1) using a sensor assembly (11) whose output signal or output data are substantially independent of a color of the calcified shell (3), wherein the image (30; 50-59) is a thermal image (50-59), and analyzing the image (30; 50-59) for detecting the position of the air cell (7), wherein analyzing the image (30; 50-59) comprises a segmentation of the image (30; 50-59), wherein the segmentation comprises an edge-oriented segmentation method or a region-oriented segmentation method, wherein a projection (32) of the air cell (7) into an image plane of the sensor assembly (11) is identified based on the fact that it has a lower average temperature; and
controlling a processing device (14) for processing the calcified shell (3) in the region of the blunt end (4) depending on the position of the air cell (7) detected by analyzing the image (30; 50-59) so that the calcified shell (3) is processed at a distance from the inner membrane (6) of the avian egg (1).

2. The method according to claim 1, wherein the segmentation of the image (30; 50-59) comprises an edge tracking algorithm, a watershed transformation, a parallel edge extraction or a sequential edge extraction.

3. The method according to claim 1, wherein the segmentation of the image (30; 50-59) comprises an energy method, region growing, region splitting, pyramid linking or split and merge.

4. The method according to any one of the preceding claims, wherein a projection of the air cell (7) into an image plane of the sensor assembly (11) is identified by the segmentation.

5. The method according to any one of the preceding claims, wherein analyzing the image (30; 50-59) comprises:
determining an area center (37) or volume center of a segment (36) of the image (30; 50-59) detected by the segmentation.

6. The method according to any one of the preceding claims, wherein a direction from which the processing device (14) acts on the calcified shell (3) is affected by controlling the processing device (14), depending on the position of the air cell (7) detected by analyzing the image (30; 50-59).

7. The method according to claim 6, wherein the processing device (14) comprises a source (15) of electromagnetic radiation and a controllable deflector (16, 17) for electromagnetic radiation generated by the source (15), wherein the deflector (16, 17) is controlled depending on the position of the air cell (7) detected by analyzing the image (30; 50-59) in order to generate a weakening line (92) in the calcified shell (3).

8. The method according to any one of the preceding claims,
wherein the sensor assembly (11) comprises an IR camera (12) for detecting a thermal image (50-59), and wherein the method comprises the step of:
using temperature information extracted from the thermal image (50-59) for quality control and/or for process control of at least one part of an incubation process of the avian egg (1).

9. A method for determining the sex of an embryo (100) contained in an incubated avian egg (1) based on a measurement at the blunt end (4) of the avian egg (1), comprising the steps of:
generating a weakening line (92) in the calcified shell (3) of the avian egg (1) with the method according to any one of the preceding claims,
lifting a region (94) of the calcified shell (3) defined by the weakening line for opening the avian egg (1),
performing at least one measurement on the opened avian egg for determining the sex of the embryo (100), and
closing the opened calcified shell (3) of the avian egg (1) by putting on a closure material (96).

10. A device (10; 80) for processing a calcified shell (3) of an avian egg (1) containing an embryo (100) therein in the region of the blunt end (4) of the avian egg (1), particularly for generating a weakening line in the calcified shell (3) in the region of the blunt end (4), comprising:
a sensor device (11) whose output signal or output data are substantially independent of a color of the calcified shell (3) for capturing an image (30; 50-59) of the avian egg (1), wherein the sensor device (11) comprises an IR camera,
a control device (13) for analyzing the image (30; 50-59) in order to detect a position of an air cell (7) which is disposed between an inner membrane (6) and an outer membrane (5) of the avian egg (1) in the region of the blunt end (4), and
a processing device (14) which is controllable by the control device (13) for processing the calcified shell (3) in the region of the blunt end (4) depending on the position of the air cell (7) detected by the control device in order to process the calcified shell (3) at a distance from the inner membrane (6) of the avian egg (1),
wherein the control device (13) is configured to segment the image (30; 50-59) using an edge-orientated segmentation method or a region-oriented segmentation method,
wherein the control device (13) is configured to identify a projection (32) of the air cell (7) into an image plane of the sensor device (11) based on the fact that it has a lower average temperature.

11. The device according to claim 10, wherein the control device (13) is configured to segment the image (30; 50-59) using an edge tracking algorithm, a watershed transformation, a parallel edge extraction or a sequential edge extraction.

12. The device according to claim 10, wherein the control device (13) is configured to segment the image (30; 50-59) using an energy method, region growing, region splitting, pyramid linking or split and merge.

13. The device according to any one of claims 10 to 12, wherein the control device (13) is configured to detect an area center (37) or volume center of a segment (36) of the image (30; 50-59) detected by the segmentation of the image (30; 50-59).

14. The device according to any one of claims 10 to 13,
wherein the processing device (14) is configured to generate a weakening line (92) in the calcified shell (3), and wherein the device (10; 80) further comprises:
an opening device (83) for lifting a region (94) of the calcified shell (3) defined by the weakening line (92) in order to open the avian egg (1),
a measurement device (85) for performing at least one measurement on the opened avian egg for determining a sex of the embryo (100), and
a closure device (86) for closing the opened calcified shell (3) of the avian egg (1).

15. The device according to any one of claims 10 to 14, wherein the device (10; 80) is configured for carrying out the method according to any one of claims 1 to 9.

## Revendications

1. Procédé de traitement d'une coquille de calcaire (3) d'un œuf d'oiseau (1) avec l'embryon (100) qu'il contient dans la zone du pôle obtus (4) de l'œuf d'oiseau (1), en particulier pour introduire une ligne de faiblesse dans la coquille de calcaire (3) dans la zone du pôle obtus (4), comportant les étapes:
détermination d'une position d'une chambre à air (7) qui est disposée entre une membrane interne (6) et une membrane externe (5) de l'œuf d'oiseau (1) dans la zone du pôle obtus (4), où la détermination de la position de la chambre à air (7) comporte:
la capture d'une image (30; 50-59) de l'œuf d'oiseau (1) à l'aide d'un dispositif capteur (11) dont le signal de sortie ou les données de sortie sont sensiblement indépendants d'une couleur de la coquille de calcaire (3), où l'image (30; 50-59) est une image thermique (50-59), et l'évaluation de l'image (30; 50-59) pour déterminer la position de la chambre à air (7), où l'évaluation de l'image (30; 50-59) comporte une segmentation de l'image (30; 50-59), où la segmentation comporte une méthode de segmentation orientée bord ou une méthode de segmentation orientée région, où une projection (32) de la chambre à air (7) dans un plan image du dispositif capteur (11) est identifiée par le fait qu'elle présente une température moyenne plus basse; et
commande d'un dispositif de traitement (14) pour traiter la coquille de calcaire (3) dans la zone du pôle obtus (4) en fonction de la position de la chambre à air (7) déterminée par l'évaluation de l'image (30; 50-59), de sorte que la coquille de calcaire (3) est traitée à distance de la membrane interne (6) de l'œuf d'oiseau (1).

2. Procédé selon la revendication 1, où la segmentation de l'image (30; 50-59) comporte un algorithme de suivi de bord, une transformation de ligne de partage des eaux, une extraction de bord parallèle ou une extraction de bord séquentielle.

3. Procédé selon la revendication 1, où la segmentation de l'image (30; 50-59) présente une méthode énergétique, region growing, region splitting, pyramid linking ou split and merge.

4. Procédé selon l'une des revendications précédentes, où une projection de la chambre à air (7) dans un plan image du dispositif capteur (11) est identifiée par la segmentation.

5. Procédé selon l'une des revendications précédentes, où l'évaluation de l'image (30; 50-59) comporte:
la détermination d'un point central de surface (37) ou d'un point central de volume d'un segment (36) de l'image (30; 50-59) déterminé par la segmentation.

6. Procédé selon l'une des revendications précédentes où, par la commande du dispositif de traitement (14), une direction depuis laquelle le dispositif de traitement (14) agit sur la coquille de calcaire (3) est influencée en fonction de la position de la chambre à air (7) déterminée par l'évaluation de l'image (30; 50-59).

7. Procédé selon la revendication 6, où le dispositif de traitement (14) comporte une source (15) de rayonnement électromagnétique et un dispositif de déviation pouvant être commandé (16, 17) pour le rayonnement électromagnétique produit par la source (15), où le dispositif de déviation (16, 17) est commandé en fonction de la position de la chambre à air (7) déterminée par l'évaluation de l'image (30; 50-59), afin d'introduire une ligne de faiblesse (92) dans la coquille de calcaire (3).

8. Procédé selon l'une des revendications précédentes,
où le dispositif capteur (11) comporte une caméra thermique (12) pour capturer une image thermique (50-59), et où le procédé comporte l'étape:
utilisation d'informations de température obtenues à partir de l'image thermique (50-59) pour l'assurance de qualité et/ou pour la conduite de processus d'au moins une section d'un processus de couvaison de l'œuf d'oiseau (1).

9. Procédé de détermination du sexe d'un embryon (100) contenu dans un œuf d'oiseau couvé (1) à l'aide d'une mesure au pôle obtus (4) de l'œuf d'oiseau (1), comportant les étapes:
introduction d'une ligne de faiblesse (92) dans la coquille de calcaire (3) de l'œuf d'oiseau (1) avec le procédé selon l'une des revendications précédentes,
soulèvement d'une zone (94) de la coquille de calcaire (3) définie par la ligne de faiblesse pour ouvrir l'œuf d'oiseau (1),
réalisation d'au moins une mesure sur l'œuf d'oiseau ouvert pour déterminer le sexe de l'embryon (100) et
fermeture de la coquille de calcaire ouverte (3) de l'œuf d'oiseau (1) par application d'un matériau de fermeture (96).

10. Dispositif (10; 80) pour traiter une coquille de calcaire (3) d'un œuf d'oiseau (1) avec l'embryon (100) qu'il contient dans la zone du pôle obtus (4) de l'œuf d'oiseau (1), en particulier pour introduire une ligne de faiblesse dans la coquille de calcaire (3) dans la zone du pôle obtus (4), comportant:
un dispositif capteur (11) dont le signal de sortie ou les données de sortie sont sensiblement indépendants d'une couleur de la coquille de calcaire (3), pour saisir une image (30; 50-59) de l'œuf d'oiseau (1), où le dispositif capteur (11) comporte une caméra thermique (12),
un dispositif de commande (13) pour évaluer l'image (30; 50-59) afin de déterminer une position d'une chambre à air (7) qui est disposée entre une membrane interne (6) et une membrane externe (5) de l'œuf d'oiseau (1) dans la zone du pôle obtus (4), et
un dispositif de traitement (14), qui peut être commandé par le dispositif de commande (13), pour traiter la coquille de calcaire (3) dans la zone du pôle obtus (4) en fonction de la position de la chambre à air (7) déterminée par le dispositif de commande, pour traiter la coquille de calcaire (3) à distance de la membrane interne (6) de l'œuf d'oiseau (1),
où le dispositif de commande (13) est configuré pour segmenter l'image (30; 50-59) au moyen d'une méthode de segmentation orientée bord ou d'une méthode de segmentation orientée région, où le dispositif de commande (13) est configuré pour identifier une projection (32) de la chambre à air (7) dans un plan image du dispositif capteur (11) par le fait qu'elle présente une température moyenne plus basse.

11. Dispositif selon la revendication 10, où le dispositif de commande (13) est configuré pour segmenter l'image (30; 50-59) au moyen d'un algorithme de suivi de bord, d'une transformation de ligne de partage des eaux, d'une extraction de bord parallèle ou d'une extraction de bord séquentielle.

12. Procédé selon la revendication 10, où le dispositif de commande (13) est configuré pour segmenter l'image (30; 50-59) à l'aide d'une méthode énergétique, region growing, region splitting, pyramid linking ou split and merge.

13. Dispositif selon l'une des revendications 10 à 12, où le dispositif de commande (13) est configuré pour déterminer un point central de surface (37) ou un point central de volume d'un segment (36) de l'image (30; 50-59) déterminé par la segmentation de l'image (30; 50-59).

14. Dispositif selon l'une des revendications 10 à 13,
où le dispositif de traitement (14) est configuré pour introduire une ligne de faiblesse (92) dans la coquille de calcaire (3), et où le dispositif (10; 80) comporte en outre:
un dispositif d'ouverture (83) pour soulever une zone (94) de la coquille de calcaire (3) définie par la ligne de faiblesse (92) pour ouvrir l'œuf d'oiseau (1),
un dispositif de mesure (85) pour réaliser au moins une mesure sur l'œuf d'oiseau ouvert pour déterminer un sexe de l'embryon (100) et
un dispositif de fermeture (86) pour fermer la coquille de calcaire ouverte (3) de l'œuf d'oiseau (1).

15. Dispositif selon l'une des revendications 10 à 14, où le dispositif (10; 80) est configuré pour mettre en oeuvre le procédé selon l'une des revendications 1 à 9.
